(19) Europäisches Patentamt
European Patent Office
Office européen des brevets

(11) EP 3 855 182 B1

(12) # EUROPEAN PATENT SPECIFICATION

(45) Date of publication and mention of the grant of the patent:
**29.04.2026 Bulletin 2026/18**

(21) Application number: **20217786.1**

(22) Date of filing: **30.12.2020**

(51) International Patent Classification (IPC):
***G01N 33/53*** *(2006.01)* ***G01N 33/543*** *(2006.01)*
***G01N 33/58*** *(2006.01)* ***G01N 33/68*** *(2006.01)*
***G01N 33/92*** *(2006.01)*

(52) Cooperative Patent Classification (CPC):
**G01N 33/53; G01N 33/543; G01N 33/58; G01N 33/6896; G01N 33/92;** G01N 2800/52

(54) **METHOD FOR OBTAINING INFORMATION ON COGNITIVE FUNCTION**

VERFAHREN ZUR GEWINNUNG VON INFORMATIONEN ÜBER KOGNITIVE FUNKTION

PROCÉDÉ PERMETTANT D'OBTENIR DES INFORMATIONS SUR LA FONCTION COGNITIVE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.01.2020 JP 2020010410**

(43) Date of publication of application:
**28.07.2021 Bulletin 2021/30**

(73) Proprietor: **SYSMEX CORPORATION**
**Kobe-shi**
**Hyogo 651-0073 (JP)**

(72) Inventors:
- **Harada, Amane**
  **Hyogo, 651-0073 (JP)**
- **Murakami, Katsuhiro**
  **Hyogo, 651-0073 (JP)**
- **Miwa, Keiko**
  **Hyogo, 651-0073 (JP)**
- **Nakhaei, Elnaz**
  **Hyogo, 651-0073 (JP)**

(74) Representative: **Hoffmann Eitle**
**Patent- und Rechtsanwälte PartmbB**
**Arabellastraße 30**
**81925 München (DE)**

(56) References cited:
**US-A1- 2017 315 112**

- **AMANE HARADA ET AL: "Cholesterol Uptake Capacity: A New Measure of HDL Functionality for Coronary Risk Assessment", THE JOURNAL OF APPLIED LABORATORY MEDICINE: AN AACC PUBLICATION, vol. 2, no. 2, 31 May 2017 (2017-05-31), pages 186 - 200, XP055665095, DOI: 10.1373/jalm.2016.022913**
- **KHALIL ABDELOUAHED ET AL: "Impairment of the ABCA1 and SR-BI-mediated cholesterol efflux pathways and HDL anti-inflammatory activity in Alzheimer's disease", MECHANISMS OF AGEING AND DEVELOPMENT, vol. 133, no. 1, 9 December 2011 (2011-12-09), pages 20 - 29, XP028888057, ISSN: 0047-6374, DOI: 10.1016/J.MAD.2011.11.008**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 855 182 B1

## Description

FIELD OF THE INVENTION

The present invention relates to a method for obtaining information about cognitive function.

BACKGROUND

In recent years, a relationship between Alzheimer's disease and an ability of lipoproteins to extract cholesterol in cerebrospinal fluid has been reported. Cerebrospinal fluid is rich in lipoproteins, including ApoE and ApoAI, among apolipoproteins, and cholesterol homeostasis in brain cells is maintain due to the lipoproteins. In Yassine H.N. et al., ABCA 1-Mediated Cholesterol Efflux Capacity to Cerebrospinal Fluid Is Reduced in Patients With Mild Cognitive Impairment and Alzheimer's Disease. J Am Heart Assoc. 2016 Feb 12; 5(2), it has been described that, in cerebrospinal fluid of patients with moderate cognitive impairment and Alzheimer's disease patients, the ability of lipoproteins to extract cholesterol via a given membrane transporter is reduced.
Document Amane Harada et al.: "Cholesterol Uptake Capacity: A New Measure of HDL Functionality for Coronary Risk Assessment", The Journal of Applied Laboratory Medicine: An AACC Publication, vol. 2, no.2, pages 186-200, relates to a cell-free assay system to evaluate the capacity of HDL to accept additional cholesterol, using fluorescently labelled cholesterol and an anti-apolipoprotein A1 antibody.
Document US10809249 B2 relates to a method for measuring the cholesterol uptake capacity of lipoproteins.
Document Khalil Abdelouahed et al.: "Impairment of the ABCA1 and SR-BI-mediated cholesterol afflux pathways and HDL anti-inflammatory activity in Alzheimer's disease", Mechanisms of Ageing and Development, vol. 133, no. 1, pages 20-29, relates to investigating the effect of Alzheimer's disease on the cholesterol efflux capacity and anti-inflammatory activity of HDL.
Collection of cerebrospinal fluid imposes a heavy burden on subjects. Therefore, an object of the present invention is to provide a new means capable of obtaining information about cognitive function using a blood sample of a subject.

SUMMARY OF THE INVENTION

The present inventors have newly found that a measured value of lipid incorporated by lipoprotein in a blood sample is an indicator of cognitive impairment, and completed the present invention. Accordingly, the present invention provides a method for obtaining information about cognitive function of a subject, including contacting lipoprotein in a blood sample of a subject with a labeled lipid and measuring the labeled lipid incorporated into the lipoprotein, in which an obtained measured value is an indicator of cognitive impairment. The present invention is defined with the appended independent claims. Advantageous embodiments are defined in the appended dependent claims.
One aspect is related to a method for obtaining information on cognitive function of a subject, comprising: contacting lipoprotein in a blood sample of a subject with a labeled lipid; and measuring the labeled lipid incorporated into the lipoprotein to obtain a measured value, wherein the measured value is an indicator of cognitive impairment.
Another aspect is related to a method for determining effectiveness of medical intervention about cognitive function. The method comprises: first contacting lipoprotein in a blood sample of a subject to whom the medical intervention is not performed with a labeled lipid and measuring the labeled lipid incorporated into the lipoprotein to obtain a first measured value; after the first contacting, second contacting lipoprotein in a blood sample of the subject to whom the medical intervention has been performed with a labeled lipid and measuring the labeled lipid incorporated into the lipoprotein to obtain a second measured value; and comparing the first measured value with the second measured value to determine effectiveness of the medical intervention.
The present invention makes it possible to obtain a measured value of a labeled lipid incorporated by lipoprotein in a blood sample as a new indicator of cognitive impairment.

BRIEF DESCRIPTION OF THE DRAWINGS

Fig. 1A is a view showing an example of an appearance of a reagent kit for obtaining information about cognitive function.
Fig. 1B is a view showing an example of an appearance of a reagent kit for obtaining information about cognitive function.
Fig. 2 is a view showing an example of an appearance of a reagent kit for obtaining information about cognitive

function.

Fig. 3 is a schematic diagram showing an example of an apparatus for determining cognitive function.

Fig. 4 is a block showing a hardware configuration of an apparatus for determining cognitive function.

Fig. 5A is a flowchart for determination using an apparatus for determining cognitive function.

Fig. 5B is a flowchart for determination using an apparatus for determining cognitive function.

Fig. 5C is a flowchart for determination using an apparatus for determining cognitive function.

Fig. 6 is a flowchart for determination using an apparatus for determining cognitive function.

Fig. 7 is a graph showing a relationship between ApoE concentration in a diluent containing a lipoprotein fraction and a measurement result of an amount of cholesterol incorporated into lipoprotein.

Fig. 8 is a graph showing the amount of cholesterol incorporated into lipoprotein in a diluent containing a lipoprotein fraction containing recombinant ApoE.

Fig. 9 is a graph showing the amount of cholesterol incorporated into lipoprotein in each serum of a subject classified according to the state of cognitive function.

Fig. 10 is a graph showing the amount of ApoE lipoprotein captured in each serum of a subject classified according to the state of cognitive function.

DETAILED DESCRIPTION OF THE PREFERRED EMBODIMENTS

[1. Method for Obtaining Information about Cognitive Function]

In the method for obtaining information on cognitive function of this embodiment (hereinafter, also simply referred to as "method"), lipoprotein in a blood sample of a subject is contacted with a labeled lipid, and the labeled lipid incorporated into the lipoprotein is measured. The amount of the labeled lipid incorporated into the lipoprotein (hereinafter, also referred to as "uptake amount") represents an ability of lipoprotein to incorporate lipid. This ability is also called uptake capacity (see, e.g., Nagano et al. J Am Heart Assoc. 2019 May 7; 8(9). This literature is incorporated herein by reference). The amount of the labeled lipid incorporated into the lipoprotein or the lipid uptake capacity of lipoprotein is used as an indicator for obtaining information about cognitive function.

(Subject)

The subject is not particularly limited. The subject may be, for example, a healthy person, a person having an abnormality in cognitive function, or a person suspected of having an abnormality. Many abnormalities of cognitive function are caused by neurological diseases such as Alzheimer's Disease. Of the cognitive impairment caused by Alzheimer's disease, mild cognitive impairment is called MCI, and cognitive impairment with further advanced disease caused by Alzheimer's disease is called Alzheimer's dementia (AD) (see McKhann, et al. Alzheimer's & Dementia 7 (2011) 263-269. This literature is incorporated herein by reference).

(Blood sample)

Examples of the blood sample include whole blood or blood components such as plasma and serum. Of these, serum is particularly preferred. The blood sample may be separated or fractionated by a known method such as ultracentrifugation or polyethylene glycol (PEG) precipitation to obtain a fraction containing a predetermined lipoprotein. In this embodiment, a fraction containing a predetermined lipoprotein prepared from a blood sample (hereinafter, also referred to as "lipoprotein fraction") can be used to measure a labeled lipid. As used herein, the term "blood sample" also includes a sample containing not only whole blood and blood components collected from a subject but also a lipoprotein fraction prepared from whole blood or blood components (sample containing lipoprotein).

Lipoproteins may be any of high density lipoprotein (HDL), low density lipoprotein (LDL), medium density lipoprotein (IDL), very low density lipoprotein (VLDL), or chylomicron (CM). HDL is lipoprotein with a density of 1.063 g/mL or more. LDL is lipoprotein with a density of 1.019 g/mL or more and less than 1.063 g/mL. IDL is lipoprotein with a density of 1.006 g/mL or more and less than 1.019 g/mL. VLDL is lipoprotein with a density of 0.95 g/mL or more and less than 1.006 g/mL. CM is lipoprotein with a density of less than 0.95 g/mL.

Lipoproteins are known to esterify and incorporate sterol. When the labeled cholesterol described later is used as the labeled lipid, a fatty acid required for esterification reaction of cholesterol with lipoprotein or a composition containing the same (for example, liposome) may be added to a blood sample.

In this embodiment, the blood sample may be diluted and used. For example, in order to adjust lipoprotein concentration, a solution obtained by diluting the blood sample can be used for the measurement described later. An aqueous medium or a sample dilution reagent can be used for diluting the sample. The aqueous medium is not particularly limited as long as it does not inhibit an uptake reaction by lipoproteins. Examples of the aqueous medium include water,

physiological saline solutions, buffer solutions, and the like. Examples of the buffer solution include phosphate buffered saline (PBS), Tris-HCl, Good buffers, and the like. The sample dilution reagent refers to a liquid containing a substance useful in measuring an uptake amount in the above aqueous medium. Examples of the substance useful for measurement include surfactants having no cyclic structure, cyclic oligosaccharides, components that bind to a lipoprotein different from the lipoprotein to be measured, blocking agents, and the like.

In this embodiment, the blood sample may be diluted in the presence of a surfactant having no cyclic structure described later. For example, the blood sample may be diluted with a solution in which the surfactant having no cyclic structure is dissolved in the aqueous medium. A lipid uptake reaction by lipoproteins is promoted by using a surfactant having no cyclic structure. The surfactant having no cyclic structure also plays the role of blocking agent. Therefore, an animal-derived protein generally used as a blocking agent in immunological measurements, such as bovine serum albumin (BSA), may not be used. By mixing the diluted blood sample with the labeled lipid, the lipoprotein and the labeled lipid come into contact in the presence of the surfactant having no cyclic structure.

In this embodiment, the blood sample may be diluted in the presence of the first additive and/or the second additive described later. The first additive refers to "a compound containing a hydrocarbon chain having at least one carbon-carbon unsaturated bond (excluding a sterol)", and the second additive refers to "a saturated aliphatic compound having no phosphodiester bond". For example, the blood sample may be diluted with a liquid containing the first additive and/or the second additive in the above aqueous medium or sample dilution reagent. By mixing the blood sample diluted as described above with the labeled lipid, the lipoprotein and the labeled lipid come into contact in the presence of the first additive and/or the second additive.

In this embodiment, the blood sample may be diluted in the presence of cyclic oligosaccharides. For example, the blood sample may be diluted with a solution in which cyclic oligosaccharides are dissolved in the aqueous medium. Examples of the cyclic oligosaccharide include cyclodextrin, hydroxypropyl cyclodextrin, and the like. The cyclic oligosaccharide functions as a blocking agent by including cholesterol derived from the blood sample.

The concentration of apolipoprotein that is a component of lipoprotein is an indicator of the concentration of lipoprotein in the blood sample. In this embodiment, the lipoprotein concentration in the blood sample may be adjusted by diluting the blood sample based on the obtained apolipoprotein concentration. The concentration of apolipoprotein can be measured by known immunoassays (for example, immunoturbidimetry). As the concentration of apolipoprotein, the concentration of ApoE is particularly preferred. The measurement of apolipoprotein is preferably performed separately from the measurement of uptake amount, by taking a part of the blood sample to use it as a measurement sample.

In this embodiment, the blood sample may be diluted in the presence of a blocking agent to prevent nonspecific adsorption of the labeled lipid. For example, the blood sample may be diluted with a sample dilution reagent containing such a blocking agent. The addition of the blocking agent suppresses, for example, nonspecific adsorption of the labeled lipid to the solid phase or capture body described later. Examples of the blocking agent for preventing nonspecific adsorption of the labeled lipid include 2-methacryloyloxyethyl phosphoryl choline type polymer. This polymer may be a homopolymer of 2-methacryloyloxyethyl phosphoryl choline or a copolymer with another monomer. As another monomer, an acrylic ester or methacrylic ester having an alkyl group having 1 to 20 carbon atoms as a hydrophobic group is preferable. The 2-methacryloyloxyethyl phosphoryl choline type polymer is commercially available. Examples thereof include a series of LIPIDURE (trademark) of NOF Corporation, and among them, LIPIDURE-BL203 is particularly preferable.

(Labeled lipid)

Examples of the labeled lipid include sterols having a labeling substance (labeled sterols), phospholipids having a labeling substance (labeled phospholipids), and the like. Hereinafter, the labeling substance possessed by the labeled lipid is also referred to as a "first label". In this embodiment, the labeled lipid is labeled sterol. Examples of the sterol include cholesterol and derivatives thereof. Examples of the cholesterol derivatives include precursors of bile acids, precursors of steroids, and the like. Specifically, 3β-hydroxy-Δ5-cholenoic acid, 24-amino-5-colen-3β-ol and the like are preferable. The measurement of uptake capacity using a labeled phospholipid is described in a paper by Edward B. N. et al. (Edward B. N. et al., Biology 2019, 8, 53; doi: 10.3390/biology 8030053).

In this embodiment, the labeled sterol is cholesterol having a labeling substance (hereinafter, also referred to as "labeled cholesterol"). A cholesterol moiety in the labeled cholesterol may have a structure of naturally occurring cholesterol, or a structure of cholesterol obtained by removing one or more methylene groups and/or methyl groups from an alkyl chain bonded to C17 position of naturally occurring cholesterol (also called norcholesterol).

It is preferable to use a labeled lipid which can be esterified by lipoproteins, since lipoproteins incorporate lipid by esterification. In general, it is considered that, for example, the labeled cholesterol is esterified by lecithin-cholesterol acyltransferase (LCAT) derived from blood contained in the sample when it is mixed with the blood sample. Methods for confirming esterification of labeled cholesterol by lipoproteins themselves are known in the art and can be routinely performed by those skilled in the art.

The first label is not particularly limited as long as it is a labeling substance that makes it possible to detect a labeled lipid incorporated into lipoprotein. The first label may be, for example, a tag that is itself to be detected, or may be a substance that generates a detectable signal (hereinafter, also referred to as a "signal generating substance").

The tag as the first label is not particularly limited as long as it does not inhibit uptake of lipid by lipoproteins, and a substance capable of specifically binding to the tag is present or obtained. Hereinafter, lipid added with a tag as the first label is also referred to as "tagged lipid". Similarly, sterol added with a tag as the first label is also referred to as "tagged sterol". Also, cholesterol added with a tag as the first label is also referred to as "tagged cholesterol". Tagged cholesterol itself is known in the art and is described, for example, in US 2017/0315112 A1.

Examples of the tagged cholesterol include tagged cholesterol represented by the following formula (I).

[Chemical Formula 1]

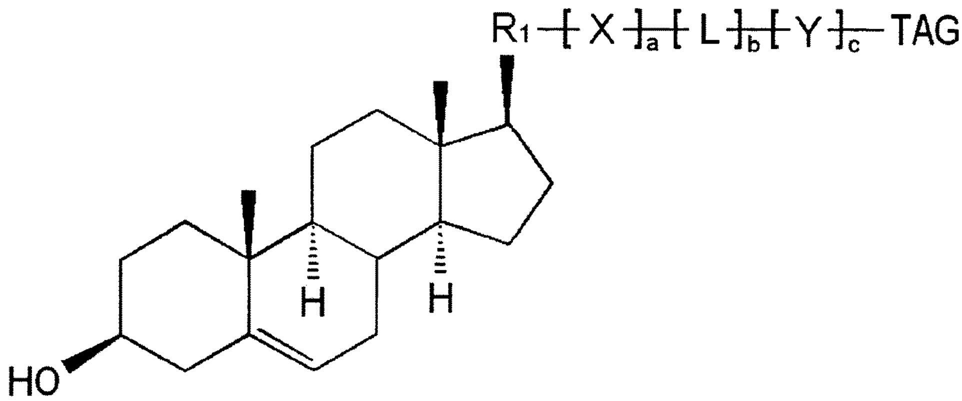

(I)

wherein $R_1$ is an alkylene group having 1 to 6 carbon atoms which optionally have a methyl group;

X and Y are identical or different, and are represented by $-R_2$-NH-, -NH-$R_2$-, - $R_2$-(C=O)-NH-, -(C=O)-NH-$R_2$-, -$R_2$-NH-(C=O)-, -NH-(C=O)-$R_2$-, -$R_2$-(C=O)-, - (C=O)-$R_2$-, -$R_2$-(C=O)-O-, -(C=O)-O-$R_2$-, -$R_2$-O-(C=O)-, -O-(C=O)-$R_2$-, -$R_2$-(C=S)-NH-, -(C=S)-NH-$R_2$-, -$R_2$-NH-(C=S)-, -NH-(C=S)-$R_2$-, -$R_2$-O-, -O-$R_2$-, -$R_2$-S-, or -S-$R_2$-, and each $R_2$ is independently an atomic bonding, an alkylene group having 1 to 10 carbon atoms which optionally have a substituent, or an arylene group or heteroarylene group having 6 to 12 carbon atoms which optionally have a substituent, or a cycloalkylene group or heterocycloalkylene group having 3 to 8 carbon atoms which optionally have a substituent;

L is represented by $-(CH_2)_d-[R_3-(CH_2)_e]_f-$ or $-[(CH_2)_e-R_3]_f-(CH_2)_d-$, and $R_3$ is an oxygen atom, a sulfur atom, -NH-, -NH-(C=O)- or -(C=O)-NH-;

TAG is a tag;

a and c are identical or different and are an integer of 0 to 6;

b is 0 or 1;

d and e are identical or different and are an integer of 0 to 12; and

f is an integer of 0 to 24.

In the formula (I), when a, b and c are all 0, the tagged cholesterol represented by the formula has no linker, and the tag and the cholesterol moiety are bonded directly. In the formula (I), when any one of a, b and c is not 0, the tagged cholesterol represented by the formula has a linker ($-[X]_a-[L]_b-[Y]_c-$) between the tag and the cholesterol moiety. It is believed that the linker further facilitates the bonding between the tag exposed on the outer surface of the lipoprotein and the capture body. Each substituent in the formula (I) will be described below.

$R_1$ may have an alkylene group having 1 to 6 carbon atoms as a main chain, and may have a methyl group at any position. $R_1$ corresponds to an alkyl chain bonded to C17 position of naturally occurring cholesterol. In this embodiment, when $R_1$ has 1 to 5 carbon atoms, $R_1$ preferably has a methyl group at the position of C20 in the naturally occurring cholesterol. When $R_1$ has 6 carbon atoms, $R_1$ preferably has the same structures as alkyl chains at C20 to C27 positions of the naturally occurring cholesterol.

$[X]_a$ corresponds to a connecting moiety between $R_1$ and L, $[Y]_c$ or the tag. $[Y]_c$ corresponds to a connecting moiety between $R_1$, $[X]_a$ or L and the tag. X and Y are determined according to the type of reaction that bonds between the cholesterol moiety and the linker and the type of reaction that bonds between the linker and the tag.

In $R_2$, the atomic bonding refers to a direct bonding without intervening any other atom. When $R_2$ is an alkylene group having 1 to 10 carbon atoms, examples of the alkylene group include methylene, ethylene, propylene, isopropylene,

butylene, isobutylene, pentylene, neopentylene, hexylene, heptylene, octylene, 2-ethylhexylene, nonylene, and decylene groups. Among them, an alkylene group having 1 to 4 carbon atoms is preferred. When $R_2$ is an alkylene group having a substituent, the above number of carbon atoms does not include the number of carbon atoms in the substituent.

When $R_2$ is an arylene group or a heteroarylene group, the group should be an aromatic ring having 6 to 12 carbon atoms which may contain one or more hetero atoms selected from N, S, O, and P. Examples of the group include phenylene, naphthylene, biphenylylene, furanylene, pyrrolene, thiophenylene, triazolene, oxadiazolene, pyridylene, and pyrimidylene groups. When $R_2$ is an arylene group or a heteroarylene group having a substituent, the above number of carbon atoms does not include the number of carbon atoms in the substituent.

When $R_2$ is a cycloalkylene group or a heterocycloalkylene group, the group should be a non-aromatic ring having 3 to 8 carbon atoms which may include one or more hetero atoms selected from N, S, O, and P. Examples of the group include cyclopropylene, cyclobutylene, cyclopentylene, cyclohexylene, cycloheptylene, cyclooctylene, pyrrolidinylene, piperidinylene, and morpholinylene groups. When $R_2$ is a cycloalkylene group or a heterocycloalkylene group having a substituent, the above number of carbon atoms does not include the number of carbon atoms in the substituent.

Examples of the substituent in $R_2$ include hydroxyl, cyano, alkoxy, =O, =S, - $NO_2$, -SH, halogen, haloalkyl, heteroalkyl, carboxyalkyl, amine, amide, and thioether groups. $R_2$ may have a plurality of the substituents. Halogen represents fluorine, chlorine, bromine, or iodine. Alkoxy represents an -O-alkyl group, and the alkyl group is a linear or branched saturated aliphatic hydrocarbon group having 1 to 5 carbon atoms, and preferably 1 or 2 carbon atoms.

Preferably, a and c are both 1, and X and Y are identical or different and are - (C=O)-NH- or -NH-(C=O)-.

L corresponds to a spacer and has a polymer structure that adds a predetermined length to the linker. It is preferable that the polymer structural moiety does not inhibit the uptake of cholesterol by lipoproteins, and that the linker moiety is hardly incorporated into lipoproteins. The polymers include hydrophilic polymers such as polyethylene glycol (PEG). In a preferred embodiment, L is a structure represented by $-(CH_2)_d-[O-(CH_2)_e]_f-$ or $-[(CH_2)_e-O]_f-(CH_2)_d-$. d and e are identical or different and are an integer of 0 to 12, preferably an integer of 2 to 6, and more preferably both 2. f is an integer of 0 to 24, preferably an integer of 2 to 11, and more preferably an integer of 4 to 11.

The tag may be any of naturally occurring substances and synthesized substances, and examples thereof include compounds, peptides, proteins, nucleic acids, a combination thereof, and the like. The compound may be a labeling compound known in the art as long as a substance capable of specifically binding to the compound is present or obtained, and examples thereof include dye compounds and the like.

It is known in the art that cholesterol is esterified to increase its lipid solubility and promote its uptake by lipoproteins. The tag attached to cholesterol may be a lipid soluble or hydrophobic substance.

Examples of combinations of a tag and a substance capable of specifically binding to the tag include an antigen and an antibody that recognizes the antigen, a hapten and an anti-hapten antibody, a peptide or a protein and an aptamer that recognizes them, a ligand and a receptor thereof, oligonucleotides and oligonucleotides having complementary strands thereof, biotin and avidin (or streptavidin), histidine tag (a peptide containing histidine of 6 to 10 residues) and nickel, glutathione-S-transferase (GST) and glutathione, and the like. The antigen as a tag may be a peptide tag and a protein tag known in the art, and examples thereof include FLAG (registered trademark), hemagglutinin (HA), Myc protein, green fluorescent protein (GFP), and the like. Examples of the hapten as a tag include 2,4-dinitrophenol and the like.

Examples of the tagged sterol include sterol added with a structure represented by a following formula (II):

[Chemical Formula 2]

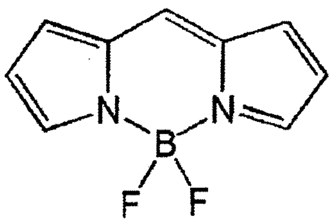

(II)

or a structure represented by a following formula (III):

[Chemical Formula 3]

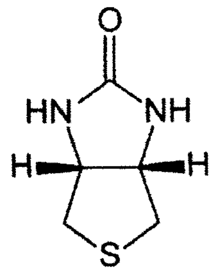

(III)

The structure represented by the formula (II) is a boron dipyrromethene (BODIPY (registered trademark)) backbone, and the structure represented by the formula (III) shows a part of biotin. Sterol added with a structure represented by the following formula (II) or (III) as a tag is preferable since capture bodies for these tags are generally available. Sterol added with a 2,4-dinitrophenyl (DNP) group as a tag is also preferable because anti-DNP antibody is commercially available.

Examples of the tagged cholesterol having no linker include fluorescently labeled cholesterol (23-(dipyrrometheneboron difluoride)-24-norcholesterol, CAS No: 878557-19-8) represented by the following formula (IV).

[Chemical Formula 4]

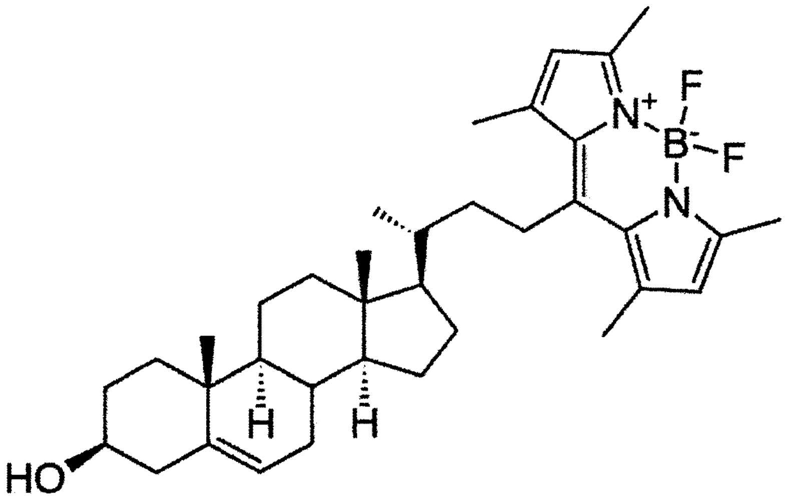

(IV)

This fluorescently labeled cholesterol is sold by Avanti Polar Lipids, Inc. under the trade name TopFluor Cholesterol. In the fluorescently labeled cholesterol represented by the formula (IV), the tag (the fluorescent moiety having BODIPY backbone structure) is directly bonded at C23 position of cholesterol. As a capture body that specifically binds to the fluorescent moiety having BODIPY backbone structure, an anti-BODIPY antibody (BODIPY FL Rabbit IgG Fraction, A-5770, Life Technologies Corporation) is commercially available.

The tagged cholesterol in which the tag is bound via a linker includes a biotin-tagged cholesterol represented by the following formula (V).

[Chemical Formula 5]

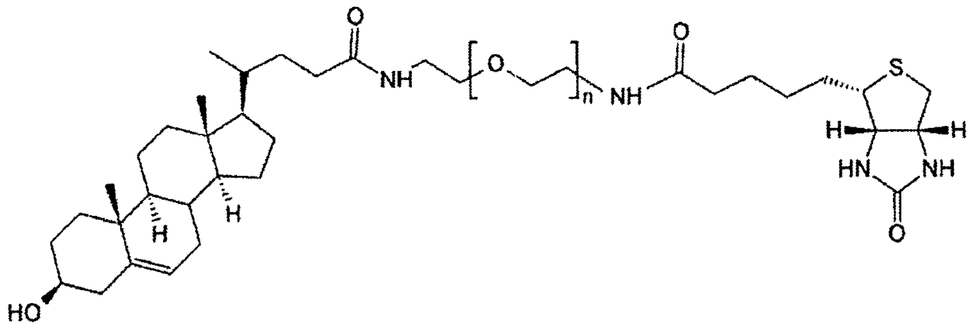

(V)

wherein, n is an integer of 0 to 24, preferably an integer of 2 to 11, and more preferably an integer of 4 to 11.

In the tagged cholesterol, the tag (the biotin moiety represented by the formula (III)) is bonded to the cholesterol moiety via the linker (polyethylene glycol). As a capture body that specifically binds to the biotin moiety, avidin or streptavidin is suitable. Avidin or streptavidin to which a labeling substance such as horseradish peroxidase (HRP) or alkaline phosphatase (ALP) is bound is also commercially available.

The tagged cholesterol in which the tag is bound via a linker includes DNP-added cholesterol represented by the following formula (VI).

[Chemical Formula 6]

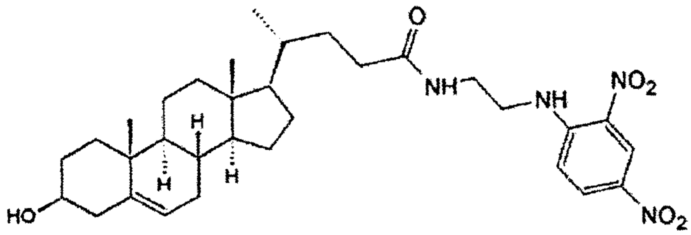

(VI)

In the tagged cholesterol, DNP is bound to the cholesterol moiety via a linker (-(C=O)-NH-$CH_2$-$CH_2$-NH-). As a capture body that specifically binds to DNP, an anti-DNP antibody is suitable. Anti-DNP antibodies to which a labeling substance such as HRP or ALP is bound is also commercially available.

Although the bonding form of the sterol moiety and the tag is not particularly limited, the sterol moiety and the tag may be bonded, or the sterol moiety and the tag may be bonded via a linker. Although the bonding means is not particularly limited, for example, a crosslink by utilizing a functional group is preferred because it is convenient. Although the functional group is not particularly limited, an amino group, a carboxyl group and a sulfhydryl group are preferred because commercially available crosslinkers can be used.

Since cholesterol has no functional group in the alkyl chain bonded to the C17 position, a cholesterol derivative having a functional group in the alkyl chain is preferably used in the addition of the tag. Examples of the cholesterol derivative include precursors of bile acid, precursor of steroids, and the like. Specifically, 3β-hydroxy-Δ5-cholenoic acid, 24-amino-5-colen-3β-ol and the like are preferable. The functional group of the tag varies depending on the type of the tag. For example, when a peptide or a protein is used as the tag, an amino group, a carboxyl group and a sulfhydryl group (SH group) can be used. When biotin is used as the tag, a carboxyl group in the side chain can be used. The linker is preferably a polymer compound having functional groups at both terminals. When biotin is added as the tag, a commercially available biotin labeling reagent may be used. The reagent contains biotin to which various length of spacer arms (for example, PEG) having a functional group such as an amino group are bound at the terminal.

Examples of the signal generating substance as the first label include fluorescent substances, luminescent substances, color-developing substances, radioactive isotopes, and the like. Among them, fluorescent substances are particularly preferred. Preferably, the fluorescent substance contains a fluorophore having a polar structure. In the art, the fluorescent substance containing a fluorophore having a polar structure itself is known.

A lipid labeled with a signal generating substance can be produced by adding the substance to a lipid by a known method. In the lipid, a position to which a signal generating substance is added is not particularly limited, and can be appropriately determined according to the type of signal generating substance to be used. Although the bonding form of the lipid and the signal generating substance is not particularly limited, it is preferable that both are directly bonded via a covalent bond.

Examples of the lipid containing a fluorescent substance include a fluorescently labeled lipid containing a fluorophore having a BODIPY backbone structure represented by the formula (II). In this embodiment, a commercially available fluorescently labeled lipid may be used. Examples of the lipid containing a fluorophore having a BODIPY backbone structure include fluorescently labeled cholesterol represented by the formula (IV). The fluorescently labeled cholesterol represented by the formula (IV) generates a fluorescence signal at a wavelength of 525 to 550 nm by an excitation wavelength of 470 to 490 nm.

(Preparing lipoprotein incorporating labeled lipid)

In this embodiment, contact between lipoprotein in a blood sample and a labeled lipid can be performed, for example, by mixing the blood sample with a solution containing the labeled lipid. After mixing them, the lipoprotein starts to incorporate the labeled lipid, and the lipoprotein incorporating the labeled lipid is obtained. In this preparing, it may be carried out in the presence of the first additive and/or the second additive described later. The order of mixing is not

particularly limited.

The conditions of temperature and time in mixing are not particularly limited. For example, a mixed solution of a blood sample and a labeled lipid may be incubated at 20 to 48°C, preferably 25 to 42°C, for 1 minute to 24 hours, preferably 10 minutes to 2 hours. During the incubation, the mixed solution may be allowed to stand, or may be stirred or shaken.

The addition amount of the labeled lipid is not particularly limited, but may be added in a slight excess so that the labeled lipid is not depleted. For example, the labeled lipid can be added to a final concentration of 0.1 μM or more and 30 μM or less, and preferably 1 μM or more and 10 μM or less.

(Surfactant having no cyclic structure)

In this embodiment, the mixing of the lipoprotein in the blood sample and the labeled lipid may be performed in the presence of a surfactant having no cyclic structure. The surfactant having no cyclic structure refers to a surfactant which is an acyclic compound. For example, a surfactant having no cyclic structure may be added to the solution containing the labeled lipid or the blood sample. The order of addition is not particularly limited. The addition amount of the surfactant having no cyclic structure can be appropriately set. For example, the concentration of the surfactant in the mixed solution obtained in the preparing the lipoprotein incorporating the labeled lipid is 0.001% (v/v) or more and 0.5% (v/v) or less, and preferably 0.01% (v/v) or more and 0.1% (v/v) or less. By performing the mixing in the presence of the surfactant having no cyclic structure, an uptake reaction by lipoprotein is promoted. The animal-derived protein may not be stable in lot-to-lot quality. The animal-derived protein tends to non-specifically bind to components in the blood sample, antibodies added to a measurement system, labeled lipids and the like, as compared to the surfactant. Since the surfactant having no cyclic structure is a synthetic product, quality is almost constant and influence on the measurement is minor compared to the animal-derived protein.

In this embodiment, the mixing of the lipoprotein in the blood sample and the labeled lipid may be performed in the presence of the cyclic oligosaccharide and surfactant having no cyclic structure. The contact between the lipoprotein in the blood sample and the labeled lipid may be performed in the presence of a component that binds to a lipoprotein different from the lipoprotein to be measured and a surfactant having no cyclic structure. For example, a component that binds to a cyclic oligosaccharide and/or a lipoprotein different from the lipoprotein to be measured may be added to the solution containing the labeled lipid or the above liquid reagent.

The surfactant having no cyclic structure can be appropriately selected from nonionic surfactants, cationic surfactants, anionic surfactants and amphoteric surfactants, but is preferably a nonionic surfactant. Nonionic surfactants having no cyclic structure are not particularly limited, and examples thereof include polyalkylene glycol ethylene oxide adduct, polyoxyethylene alkyl ether, polyoxyethylene polyoxypropylene alkyl ether, polyoxyalkylene dialkyl ether, polyoxyalkylene alkenyl ether, polyoxyethylene branched alkyl ether, polyoxyethylene polyoxypropylene alkyl ether, polyoxyalkylene ether, polyethylene glycol fatty acid ester, glycerin fatty acid ester, polyglycerol fatty acid ester, propylene glycol fatty acid ester, polypropylene glycol fatty acid ester, polyoxyethylene coconut fatty acid glyceryl, polyoxyethylene hardened castor oil, polyoxyethylene castor oil, polyoxyethylene triisostearic acid, polyoxyethylene sorbitol fatty acid ester, polyoxyethylene alkylamine, polyoxyethylene polyoxypropylene alkylamine, polyoxyethylene alkyl propylene diamine, fatty acid diethanolamide, polyoxyethylene fatty acid monoethanolamide, and the like. The nonionic surfactant having no cyclic structure may be used alone or in combination of two or more.

Among the nonionic surfactants, polyoxyethylene alkyl ether and polyalkylene glycol ethylene oxide adduct are preferable. Examples of the polyoxyethylene alkyl ether include polyoxyethylene lauryl ether, polyoxyethylene cetyl ether, polyoxyethylene stearyl ether, polyoxyethylene behenyl ether, and the like. In this embodiment, polyoxyethylene lauryl ether is particularly preferred. Polyoxyethylene lauryl ether is commercially available, and examples thereof include NONION K-230 and NONION K-220 of NOF Corporation, EMULGEN 123P and EMULGEN 130K of Kao Corporation, and the like.

In this embodiment, as the polyalkylene glycol ethylene oxide adduct, a block copolymer is preferable, and a triblock copolymer of ethylene oxide and propylene oxide is more preferable. As a nonionic surfactant of the block copolymer, a compound represented by the following formula (VII) is particularly preferable.

[Chemical Formula 7]

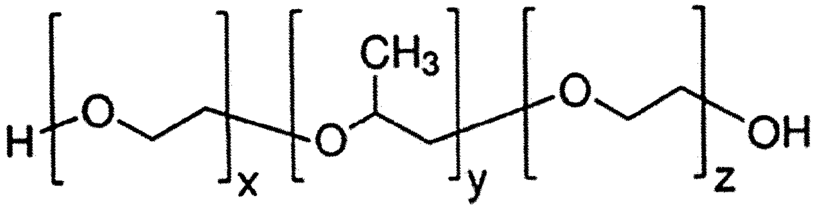

(VII)

wherein x, y and z are identical or different and an integer of 1 or more, and the molecular weight of the polypropylene oxide moiety is 2750 or less.

The compounds of the formula (VII) are also called polypropylene glycol ethylene oxide adducts, polyoxyethylene polyoxypropylene glycols, polyoxyethylene polyoxypropylene block polymers, or pluronic (trademark)-type nonionic surfactants.

In the formula (VII), the molecular weight of the polypropylene oxide moiety (hereinafter also referred to as "PPO") refers to a molecular weight of a moiety represented by $-(O-CH(CH_3)-CH_2)_y-$, and a molecular weight calculated from the molecular structural formula. In this embodiment, the molecular weight of PPO is preferably 900 or more and 2750 or less, more preferably 950 or more and 2750 or less, and particularly preferably 950 or more and 2250 or less. Because PPO in pluronic-type nonionic surfactants is relatively highly hydrophobic, the larger the molecular weight of PPO, the greater the hydrophobicity of the surfactant. Therefore, a pluronic-type nonionic surfactant having a molecular weight of PPO more than 2750 may act on lipoprotein to inhibit uptake of lipids.

The average molecular weight of the compound represented by the formula (VII) is preferably 1000 to 13000. In the formula (VII), the sum of x and z is preferably 2 to 240. In the formula (VII), y is preferably 15 to 47, more preferably 16 to 47, and particularly preferably 16 to 39. As used herein, the "average molecular weight" is a weight average molecular weight measured by gel permeation chromatography (GPC).

Pluronic-type nonionic surfactants are commercially available, and examples thereof include the Pluronic series of BASF, the Pronon series of NOF Corporation, the Adeka Pluronic series of Asahi Denka Co., Ltd., and the like. The Pluronic series is classified by a Pluronic grid in which the molecular weight of PPO is taken on the vertical axis and the ethylene oxide (EO) content (%) is taken on the horizontal axis (for example, see Pitto-Barry A. and Barry N.P.E., Poly. Chem., 2014, vol. 5, p. 3291-3297). The Pluronic grid is provided not only from academic literature but also from various manufacturers. In this embodiment, the compound represented by the formula (VII) may be selected based on the pluronic grid.

Examples of the pluronic-type nonionic surfactants represented by the formula (VII) include Pluronic L31 (molecular weight of PPO: 950, EO content: 10%), Pluronic L35 (molecular weight of PPO: 950, EO content: 50%), Pluronic F38 (molecular weight of PPO: 950, EO content: 80%), Pluronic L42 (molecular weight of PPO: 1200, EO content: 20%), Pluronic L43 (molecular weight of PPO: 1200, EO content: 30%), Pluronic L44 (molecular weight of PPO: 1200, EO content: 40%), Pluronic L61 (molecular weight of PPO: 1750, EO content: 10%), Pluronic L62 (molecular weight of PPO: 1750, EO content: 20%), Pluronic L63 (molecular weight of PPO: 1750, EO content: 30%), Pluronic L64 (molecular weight of PPO: 1750, EO content: 40%), Pluronic P65 (molecular weight of PPO: 1750, EO content: 50%), Pluronic F68 (molecular weight of PPO: 1750, EO content: 80%), Pluronic L72 (molecular weight of PPO: 2050, EO content: 20%), Pluronic P75 (molecular weight of PPO: 2050, EO content: 50%), Pluronic F77 (molecular weight of PPO: 2050, EO content: 70%), Pluronic L81 (molecular weight of PPO: 2250, EO content: 10%), Pluronic P84 (molecular weight of PPO: 2250, EO content: 40%), Pluronic P85 (molecular weight of PPO: 2250, EO content: 50%), Pluronic F87 (molecular weight of PPO: 2250, EO content: 70%), Pluronic F88 (molecular weight of PPO: 2250, EO content: 80%), Pluronic L92 (molecular weight of PPO: 2750, EO content: 20%), Pluronic P94 (molecular weight of PPO: 2750, EO content: 40%), Pluronic F98 (molecular weight of PPO: 2750, EO content: 80%), and the like.

Alternatively, the triblock copolymer of ethylene oxide and propylene oxide may be a compound represented by the following formula (VIII). This compound is also called a reverse pluronic-type nonionic surfactant.

[Chemical Formula 8]

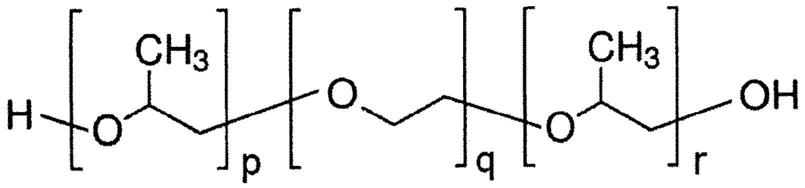 (VIII)

wherein p, q and r are identical or different and an integer of 1 or more, and the molecular weight of the polypropylene oxide moiety is 2750 or less.

In the formula (VIII), the molecular weight of the polypropylene oxide moiety refers to a molecular weight of a moiety represented by $-(O-CH(CH_3)-CH_2)_p-$ and $-(O-CH(CH_3)-CH_2)_r-$, and a molecular weight calculated from the molecular structural formula. In this embodiment, the molecular weight of PPO is preferably 900 or more and 2750 or less, and more preferably 950 or more and 2250 or less. Pluronic-type nonionic surfactants represented by the formula (VIII) are commercially available, and examples thereof include Pluronic 10R5 (molecular weight of PPO: 950, EO content: 50%) of BASF and the like.

The average molecular weight of the compound represented by the formula (VIII) is preferably 1000 to 13000. In the formula (VIII), q is preferably 2 to 240. In the formula (VIII), the sum of p and r is preferably 15 to 47, more preferably 16 to 47, and particularly preferably 16 to 39.

In the method of this embodiment, the measurement of uptake amount may be performed, when the lipoprotein in the blood sample is contacted with the labeled lipid, in the presence of a compound containing a hydrocarbon chain having at least one carbon-carbon unsaturated bond (excluding a sterol), and/or a saturated aliphatic compound having no phosphodiester bond.

Hereinafter, the "compound containing a hydrocarbon chain having at least one carbon-carbon unsaturated bond (excluding a sterol)" is also called "first additive". The "saturated aliphatic compound having no phosphodiester bond" is also called "second additive". By mixing the lipoprotein in the sample and the labeled lipid in the presence of these additives, variation of measured values of the uptake amount is reduced as compared with a case where no additive is used. That is, by using the first additive and/or the second additive, it is possible to improve reproducibility of the measurement of uptake amount.

(Compound containing hydrocarbon chain having at least one carbon-carbon unsaturated bond)

In the first additive, the hydrocarbon chain having at least one carbon-carbon unsaturated bond is a site having a chain structure having a terminal and does not form a ring in a compound molecule. The hydrocarbon chain having at least one carbon-carbon unsaturated bond may have a cyclic structure in the chain as long as it is a chain structure having a terminal. Examples of the cyclic structure include non-aromatic rings having 3 to 8 carbon atoms and aromatic rings having 6 to 12 carbon atoms. Examples of the non-aromatic ring include cyclic hydrocarbons, cyclic ethers, cyclic esters (lactones), and the like. Examples of the aromatic ring include a benzene nucleus, a naphthalene nucleus, and the like. The cyclic structure may contain one or more heteroatoms selected from N, S, O and P. However, the first additive is a compound other than sterols. For example, cholesterol, the labeled sterol used in the method of this embodiment and the like are excluded from the first additive. In a preferred embodiment, the first additive is a chain compound.

The hydrocarbon chain having at least one carbon-carbon unsaturated bond may be linear or branched. In this embodiment, a main chain of the hydrocarbon chain having at least one carbon-carbon unsaturated bond has, for example, 8 or more and 30 or less carbon atoms, preferably 10 or more and 26 or less carbon atoms, and more preferably 14 or more and 22 or less carbon atoms. The main chain of the hydrocarbon chain having at least one carbon-carbon unsaturated bond refers to a chain having a largest number of carbons, which is determined when naming the first additive according to International Union of Pure and Applied Chemistry (IUPAC) nomenclature.

In the first additive, the number of carbon-carbon unsaturated bonds contained in the hydrocarbon chain may be one or plural. The carbon-carbon unsaturated bond may be a double bond or a triple bond. When there are multiple carbon-carbon unsaturated bonds, the hydrocarbon chain of the first additive may contain both double and triple bonds.

The first additive preferably has at least one hydrophilic group. A position of the hydrophilic group is not particularly limited, but it is preferable that the hydrocarbon chain having at least one carbon-carbon unsaturated bond has the hydrophilic group. The hydrophilic group may be contained in either a main chain or a side chain of the hydrocarbon chain. Examples of the hydrophilic group include an amide group, a hydroxyl group, an amino group, a carboxyl group, a sulfo group, a thiol group, a carbonyl group, an ester group, an ether group, and a combination thereof. The first additive may be, for example, a compound represented by a following formula (IX).

$R_4$-CH=CH-$R_5$ ... (IX)

wherein,

$R_4$ and $R_5$ are identical or different and are -$R_6$-(C=O)-$NH_2$, -$R_6$-OH, -$R_6$-$NH_2$, -$R_6$-(C=O)-OH, -$R_6$-$SO_2$-OH, -$R_6$-SH, -$R_6$-O-(C=O)-OH, -$R_6$-NH-(C=O)-$NH_2$, a substituted or unsubstituted alkyl group whose main chain has 1 or more and 28 or less carbon atoms, or a hydrogen atom, provided that when $R_4$ is an alkyl group or a hydrogen atom, $R_5$ is other than the alkyl group and the hydrogen atom,
the sum of carbon numbers of main chains of $R_4$ and $R_5$ is 6 or more and 28 or less, and
$R_6$ is an atomic bonding, or a substituted or unsubstituted alkylene group whose main chain has 1 or more and 28 or less carbon atoms.

In $R_4$ and $R_5$, the substituted or unsubstituted alkyl group whose main chain has 1 or more and 28 or less carbon atoms may have a branched chain (side chain). The main chain refers to a longest carbon chain in the above alkyl group. In the above alkyl group, the main chain having 1 or more and 28 or less carbon atoms refers to a moiety designated as a linear alkyl group selected from the group consisting of methyl, ethyl, propyl, butyl, pentyl, hexyl, heptylene, octyl, nonyl, decyl, undecyl, dodecyl, tridecyl, tetradecyl, pentadecyl, hexadecyl, heptadecyl, octadecyl, nonadecyl, icosyl, henicosyl, docosyl, tricosyl, tetracosyl, pentacosyl, hexacosyl, heptacosyl, and octacosyl. When $R_4$ and/or $R_5$ is a substituted alkyl group whose main chain has 1 or more and 28 or less carbon atoms, a substituent is selected so that a linear carbon chain having 1 or more and 28 or less carbon atoms becomes a main chain.

In $R_6$, the atomic bonding refers to a direct bonding without intervening any other atom. In $R_6$, the substituted or unsubstituted alkylene group whose main chain has 1 or more and 28 or less carbon atoms may have a branched chain (side chain). The main chain refers to a longest carbon chain in the above alkylene group. In the above alkylene group, the main chain having 1 or more and 28 or less carbon atoms refers to a moiety designated as a linear alkylene group selected from the group consisting of methylene, ethylene, propylene, butylene, pentylene, hexylene, heptylene, octylene, nonylene, decylene, undecylene, dodecylene, tridecylene, tetradecylene, pentadecylene, hexadecylene, heptadecylene, octadecylene, nonadecylene, icosylene, henicosylene, docosylene, tricosylene, tetracosylene, pentacosylene, hexacosylene, heptacosylene, heptacosylene, and octacosylene. When $R_6$ is a substituted alkylene group whose main chain has 1 or more and 28 or less carbon atoms, a substituent is selected so that a linear carbon chain having 1 or more and 28 or less carbon atoms becomes a main chain.

Examples of the substituent in $R_4$, $R_5$ and $R_6$ include a cyano group, an alkoxy group, =O, =S, -$NO_2$, -SH, halogen, a haloalkyl group, a heteroalkyl group, a carboxyalkyl group, a thioether group, and the like. Each of $R_4$, $R_5$ and $R_6$ may have a plurality of substituents. Halogen represents fluorine, chlorine, bromine, or iodine. Alkoxy represents an -O-alkyl group. The alkyl group in the substituent is a linear or branched saturated aliphatic hydrocarbon group having 1 or more and 5 or less carbon atoms, and preferably 1 or 2 carbon atoms.

The compound represented by the formula (IX) may be in cis form or trans form. Examples of the compound represented by the formula (IX) include linear unsaturated aliphatic amides, unsaturated aliphatic alcohols and unsaturated aliphatic amines having one carbon-carbon double bond and having 8 or more and 30 or less carbon atoms. Specific examples thereof include cis-4-decenoic acid amide, palmitoleic acid amide, oleic acid amide, elaidic acid amide, erucic acid amide, cis-4-decen-1-ol, palmitoleyl alcohol, oleyl alcohol, elaidyl alcohol, erucyl alcohol, cis-4-decenamine, palmitoleylamine, oleylamine, trans-9-octadecenamine, cis-13-docosenamine, and the like. Among them, oleic acid amide, erucic acid amide, oleyl alcohol, palmitoleyl alcohol, cis-4-decen-1-ol, elaidyl alcohol and oleylamine are preferred.

(Saturated aliphatic compound having no phosphodiester bond)

The second additive is an aliphatic compound having no phosphodiester bond and having no carbon-carbon unsaturated bond. The aliphatic compound refers to a compound other than aromatic compounds. Since the second additive has no phosphodiester bond, for example, phospholipids, saturated aliphatic compounds having a structure similar to phospholipids and the like are excluded from the second additive. The second additive may be a chain compound having no cyclic structure in the molecule or a cyclic compound. Examples of the cyclic structure include a non-aromatic ring having 3 to 8 carbon atoms. Examples of the non-aromatic ring include cyclic hydrocarbons, cyclic ethers, lactones, and the like. The cyclic structure may contain one or more heteroatoms selected from N, S, O and P.

As the second additive, a saturated aliphatic compound whose main chain has 8 or more and 30 or less carbon atoms is preferred. The main chain of the saturated aliphatic compound as the second additive refers to a chain having a largest number of carbons, which is determined when naming the second additive according to the IUPAC nomenclature. Examples of the saturated aliphatic compound include saturated aliphatic amides, saturated aliphatic alcohols and saturated aliphatic amines having 8 or more and 30 or less carbon atoms in the main chain. The preferred second additive

is a linear saturated aliphatic amide, saturated aliphatic alcohol or saturated aliphatic amine having 8 or more and 30 or less carbon atoms.

Examples of the linear saturated aliphatic amide having 8 or more and 30 or less carbon atoms include octanoic acid amide, nonanoic acid amide, decanoic acid amide, undecanoic acid amide, dodecanoic acid amide, tetradecanoic acid amide, hexadecanoic acid amide (palmitic acid amide), octadecanoic acid amide (stearic acid amide), icosanoic acid amide, docosanoic acid amide, tetracosanoic acid amide, hexacosanoic acid amide, octacosanoic acid amide, triacontanic acid amide, and the like. Examples of the linear saturated aliphatic alcohol having 8 or more and 30 or less carbon atoms include n-octanol, n-nonanol, n-decanol, n-undecanol, n-dodecanol, n-tetradecanol, n-hexadecanol, n-octadecanol, n-icosanol, n-docosanol, n-tetracosanol, n-hexacosanol, n-octacosanol, n-triacontanol, and the like. Examples of the linear saturated aliphatic amine having 8 or more and 30 or less carbon atoms include octylamine, nonylamine, decylamine, undecylamine, dodecylamine, tetradecylamine, hexadecylamine, octadecylamine, icosylamine, docosylamine, tetracosylamine, hexacosylamine, octacosylamine, triacontylamine, and the like. Preferred second additives are stearic acid amide and palmitic acid amide.

The addition amount of the first additive and/or the second additive can be appropriately determined. For example, the concentration of the first additive and/or the second additive in the mixed solution obtained in the preparation of the lipoprotein incorporating the labeled lipid is 1 $\mu$M or more and 1000 $\mu$M or less, and preferably 13 $\mu$M or more and 352 $\mu$M or less. The mixed solution obtained in the preparation is a liquid containing the lipoprotein, the labeled lipid, and the first additive and/or the second additive. The mixed solution is, for example, a mixed solution of a sample containing lipoprotein, a solution containing a labeled lipid, and a liquid reagent containing a first additive and/or a second additive. Alternatively, the mixed solution may be a mixed solution of a sample containing lipoprotein and a liquid reagent containing a first additive and/or a second additive and a labeled lipid. When the concentration of the first additive and/or the second additive in the mixed solution is within the above range, variation of measured values is further reduced.

(Formation of complex of lipoprotein and first capture body)

In the method of this embodiment, it is included contacting lipoprotein incorporating a labeled lipid with a capture body (first capture body) that binds to the lipoprotein to form a complex containing the lipoprotein incorporating the labeled lipid and the capture body. The contact between the lipoprotein and the first capture body causes the first capture body to bind to the lipoprotein to form a complex of the lipoprotein and the first capture body.

The first capture body is not particularly limited as long as it is a substance capable of specifically binding to a part of the surface of lipoprotein. As the first capture body, an antibody that specifically binds to lipoprotein is preferable, and an antibody that can specifically bind to apolipoprotein that is a component of lipoprotein is more preferable. Examples of such antibodies include anti-ApoA antibodies (anti-ApoAI antibody and anti-ApoAII antibody), anti-ApoB antibody, anti-ApoE antibodies (anti-ApoE2 antibody, anti-ApoE3 antibody and anti-ApoE4 antibody), and the like. According to the claimed invention, the capture body is an anti-ApoE antibody. Commercially available anti-lipoprotein antibodies and anti-ApoE antibodies may be used.

In the art, it is known that genotype of lipoprotein ApoE lowers an ability to extract cholesterol from cells in the order of ApoE2 > ApoE3 > ApoE4 (see J Neurochem. 2000 Mar: 74 (3): 1008-16. This literature is incorporated herein by reference). Regarding the uptake amount, the genotype of ApoE is one factor that determines the uptake amount, and it is considered that the uptake amount is related to various factors such as post-translational modification. In this embodiment, since the amount of the labeled lipid actually taken up is measured as the uptake amount, it is considered that determination of cognitive function can be made with higher accuracy than determination of only innate genotype.

In the measurement system of the above literature, apolipoprotein is acted on cells to measure the amount of cholesterol extracted from the cells. On the other hand, in this embodiment, the labeled lipid is directly incorporated into lipoprotein in the blood sample, and the amount of incorporated labeled lipid is measured. Accordingly, the method of this embodiment does not require cells for measurement and can be performed in a cell-free system. The cell-free system means that cells are not added for the purpose of being used to measure the uptake amount. That is, the method of this embodiment can be carried out without adding cells for measurement and using their properties and functions, and the like. In this embodiment, even when a sample to be used contains cells derived from a subject, it is considered that the cells themselves hardly affect the lipoprotein's ability to uptake labeled lipid, and the measurement is considered to be cell-free system. In this embodiment, each measurement is performed in a substantially cell-free system. The substantially cell-free system means that cells are not positively added for the purpose of being used to measure the uptake amount. In this embodiment, difference in the genotype of lipoprotein ApoE can be evaluated by using the anti-ApoE2 antibody, the anti-ApoE3 antibody and the anti-ApoE4 antibody properly and measuring the uptake amount in a cell-free system.

The anti-lipoprotein antibodies and the anti-ApoE antibodies may be monoclonal antibodies or polyclonal antibodies. The origin of the antibody is not particularly limited, and may be an antibody derived from any mammal such as a mouse, a rat, a hamster, a rabbit, a goat, a horse, or a camel. The isotype of antibody may be any of IgG, IgM, IgE, IgA

and the like and is preferably IgG. In this embodiment, a fragment of an antibody and a derivative thereof may be used as the first capture body, and examples thereof include Fab fragments, F(ab')2 fragments, and the like.

The contact of the lipoprotein incorporating the labeled lipid with the first capture body may be performed, for example, by mixing a sample containing lipoprotein, a solution containing the labeled lipid, and a solution containing the first capture body, in the presence of the first additive and/or the second additive. The order of mixing is not particularly limited. The mixing may be performed in the presence of a surfactant having no cyclic structure. In a preferred embodiment, the lipoprotein and the labeled lipid are mixed in the presence of the first additive and/or the second additive, and then the mixing of the lipoprotein and the first capture body is performed. For example, the sample containing lipoprotein and the solution containing the labeled lipid are mixed in the presence of the first additive and/or the second additive, and then the obtained mixed solution and the solution containing the first capture body are mixed. Mixing in the presence of the first additive and/or the second additive is preferably performed using the above liquid reagent. As a result, a complex of the lipoprotein incorporating the labeled lipid and the first capture body is formed. The addition amount of the first capture body is not particularly limited, and can be appropriately set by those skilled in the art according to the type of the first capture body and the like.

There are no particular limitations on the conditions of temperature and time in the contact between the lipoprotein and the first capture body. For example, the mixed solution may be incubated at 20 to 48°C, preferably 25 to 42°C, for 10 seconds to 24 hours, and preferably 1 minute to 2 hours. During the incubation, the mixed solution may be allowed to stand, or may be stirred or shaken.

(Immobilization of complex on solid phase)

In this embodiment, the complex of the lipoprotein incorporating the labeled lipid and the first capture body may be immobilized on a solid phase. For example, a blood sample, a labeled lipid solution, a solution containing the first capture body, and a solid phase may be mixed. Immobilization may be performed by mixing a sample containing lipoprotein, a solution containing the labeled lipid, a solution containing the first capture body and a solid, in the presence of the first additive and/or the second additive. Alternatively, the sample containing lipoprotein, the solution containing the labeled lipid, and the solution containing the first capture body are mixed, in the presence of the first additive and/or the second additive, and then the obtained mixed solution and a solid phase may be mixed. Preferably, first, the sample containing lipoprotein and the solution containing the labeled lipid are mixed, subsequently the obtained mixed solution and the solution containing the first capture body are mixed, and then the obtained mixed solution and the solid phase may be mixed, in the presence of the first additive and/or the second additive.

In this embodiment, the first capture body may be previously immobilized on a solid phase. For example, a complex is formed on a solid phase by adding a solid phase on which an anti-lipoprotein antibody is immobilized to the mixed solution of the blood sample and the labeled lipid solution. Alternatively, the sample containing lipoprotein, the solution containing the labeled lipid, and a solid phase on which the first capture body is immobilized may be mixed, in the presence of the first additive and/or the second additive, and. Preferably, the sample containing lipoprotein and the solution containing the labeled lipid are mixed, and then the obtained mixed solution and the solid phase on which the first capture body is immobilized may be mixed, in the presence of the first additive and/or the second additive. The mixing may be performed in the presence of a surfactant having no cyclic structure. The conditions of temperature and time when using a solid phase are not particularly limited. For example, the conditions may be similar to those for the contact between the lipoprotein and the first capture body.

The solid phase is preferably a solid phase capable of capturing the first capture body in the complex. The type of the solid phase is not particularly limited, and examples of the solid phase include a solid phase of a material that physically adsorbs the antibody, a solid phase on which a molecule that specifically binds to the antibody is immobilized, and the like. Examples of the molecule that specifically binds to the antibody include protein A or G, an antibody (i.e., a secondary antibody) that specifically recognizes an antibody, and the like. A combination of substances interposed between the antibody and the solid phase can be used to bind them. Examples of such a combination of substances include combinations of biotin and avidin (or streptavidin), haptens and anti-hapten antibodies, and the like. For example, when the first capture body is previously modified with biotin, the first capture body can be captured by a solid phase on which avidin or streptavidin is immobilized.

The solid phase material can be selected from organic polymer compounds, inorganic compounds, biopolymers, and the like. Examples of the organic polymer compounds include latex, polystyrene, polypropylene, styrene-methacrylic acid copolymer, styrene-glycidyl (meth)acrylate copolymer, styrene-styrene sulfonate copolymer, methacrylic acid polymer, acrylic acid polymer, acrylonitrile butadiene styrene copolymer, vinyl chloride-acrylate copolymer, polyvinyl acetate acrylate, and the like. Examples of the inorganic compounds include magnetic bodies (iron oxide, chromium oxide, cobalt, ferrite, etc.), silica, alumina, glass, and the like. Examples of the biopolymer include insoluble agarose, insoluble dextran, gelatin, cellulose, and the like. Two or more of these may be used in combination.

The shape of the solid phase is not particularly limited, and examples thereof include a particle, a microplate, a

microtube, a test tube, and the like. Among them, microplates and particles are preferred, and 96-well microplates and magnetic particles are particularly preferred. When the shape of the solid phase is a particle, a suspension of the particles can be used for the above mixing as the solid phase. When the shape of the solid phase is a container such as a microplate, the above mixing can be performed in the container as the solid phase.

When a suspension of the particles is used as the solid phase, and the first additive and/or the second additive are further used, the concentration of the first additive and/or the second additive in the mixed solution containing the solid phase obtained in the forming a complex is 1 μM or more and 1000 μM or less, and preferably 10 μM or more and 271 μM or less. When the solid phase is a particle, the mixed solution containing the solid phase obtained in the forming a complex is a liquid containing lipoprotein, a labeled lipid, a first capture body, and particles. When using the second capture body, the mixed solution containing the solid phase obtained in the forming a complex is a liquid containing lipoprotein, a labeled lipid, a first capture body, a second capture body described later, and particles. When the solid phase is a container, the mixed solution containing the solid phase obtained in the forming a complex is a liquid containing lipoprotein, a labeled lipid, and a first capture body in the container. When using the second capture body, the mixed solution containing the solid phase obtained in the forming a complex is a liquid containing lipoprotein, a labeled lipid, a first capture body, and a second capture body in the container. These mixed solutions may contain a first additive and/or a second additive.

The mixed solution containing the solid phase obtained in the forming a complex is, specifically, a mixed solution of a sample containing lipoprotein, a liquid reagent containing a solution containing a labeled lipid, a solution containing a first capture body, and a suspension of particles. When using the second capture body, the mixed solution is a mixed solution of a sample containing lipoprotein, a liquid reagent containing a solution containing a labeled lipid, a solution containing a first capture body, a solution containing a second capture body, and a suspension of particles. The liquid reagent may be a reagent containing a labeled lipid. A first additive and/or a second additive may be contained in the mixed solution containing the solid phase obtained in the forming a complex. When the concentration of the first additive and/or the second additive is within the above range, variation of measured values can be reduced.

(Washing)

In this embodiment, a washing step of removing unreacted free components may be performed between the contact between the lipoprotein and the labeled lipid and the measurement of uptake amount described later. This washing includes B/F separation and washing of the complex. The unreacted free components are components that do not constitute a complex containing lipoprotein incorporating a labeled lipid. Examples thereof include a free labeled lipid that has not been incorporated into lipoprotein, a free first capture body that has not bound to lipoprotein, and the like. The means for B/F separation is not particularly limited, but the complex and the unreacted free components can be separated by recovering only the complex by, for example, ultracentrifugation. In a case where the complex is formed on a solid phase, when the solid phase is particles, the complex and the unreacted free components can be separated by recovering the particles by centrifugation or magnetic separation, and removing the supernatant. When the solid phase is a container such as a microplate or a microtube, the complex and the unreacted free component can be separated by removing a liquid containing the unreacted free component.

After removing the unreacted free components, the recovered complex can be washed with a suitable aqueous medium. Examples of the aqueous medium include water, physiological saline, PBS and Tris-HCl, Good buffers, and the like. In this embodiment, the washing step is preferably performed in the presence of a surfactant having no cyclic structure. For example, a surfactant having no cyclic structure may be dissolved in the aqueous medium to prepare a washing solution, and the recovered complex may be washed with the washing solution. When a solid phase is used, the solid phase that has captured the complex may be washed with a washing solution. Specifically, a washing solution is added to the recovered complex or the solid phase that has captured the complex, and B/F separation is performed again.

(Measurement of uptake amount)

In the method of this embodiment, after preparing the lipoprotein incorporating the labeled lipid, the amount of the labeled lipid incorporated into the lipoprotein is measured (hereinafter, also referred to as "measuring of uptake amount"). Measuring of uptake amount is performed by detecting a signal derived from the labeled lipid incorporated into the lipoprotein. Since this signal reflects the amount of labeled lipid incorporated into the lipoprotein, a detection result of the signal is as an indicator of uptake amount.

The phrase "detecting a signal" herein includes qualitatively detecting the presence or absence of a signal, quantifying a signal intensity, and semi-quantitatively detecting the intensity of a signal in a plurality of stages, such as "not generating signal", "weak", and "strong". In this embodiment, it is preferable to quantify the signal intensity to obtain a measured value.

The signal derived from the labeled lipid incorporated into the lipoprotein may be a signal directly generated from the labeled lipid. The signal can be detected, for example, when using a labeled lipid having a signal generating substance

as the first label. That is, the uptake amount can be measured by detecting the signal generated from the first label in the labeled lipid incorporated into the lipoprotein. For example, in the case of using a fluorescently labeled lipid, a fluorescence intensity should be measured. Methods for measuring fluorescence intensity themselves are known in the art. For example, a fluorescence intensity generated from the complex can be measured by using known measuring devices such as a spectrofluorimeter and a fluorescence plate reader. The excitation wavelength and the fluorescence wavelength can be appropriately determined according to the type of fluorescent labeled lipid used. For example, when the fluorescently labeled cholesterol of the formula (IV) is used, the excitation wavelength should be determined from the range of 470 to 490 nm, and the fluorescence wavelength should be determined from the range of 525 to 550 nm.

(Formation of complex of labeled lipid and second capture body)

The signal derived from the labeled lipid incorporated into the lipoprotein may be a signal generated when detecting the labeled lipid incorporated into the lipoprotein. In order to detect the labeled lipid incorporated into the lipoprotein, in this embodiment, the labeled lipid may be contacted with a second capture body that binds to the labeled lipid to form a complex. In this case, the labeled lipid is preferably labeled sterol, and more preferably tagged sterol. The second capture body is preferably a substance that specifically binds to the tag.

The detection principle of the tagged cholesterol incorporated into lipoprotein is as follows. It is considered that when cholesterol is incorporated into lipoprotein, the cholesterol normally migrates from a surface layer to a central part of the lipoprotein particle. In the tagged cholesterol, it is the cholesterol moiety that is incorporated into lipoprotein, and the tag is considered to be exposed on an outer surface of the lipoprotein. The "outer surface of the lipoprotein" refers to the outer surface of the lipoprotein particles. The "exposed on an outer surface" means that both existing on the outer surface of the lipoprotein and protruding from the outer surface of the lipoprotein. In this embodiment, the tag exposed on the outer surface is brought into contact with a second capture body that specifically binds to the tag to form a complex. Then, cholesterol incorporated into the lipoprotein is detected by detecting the second capture body in this complex.

The substance that specifically binds to the tag can be appropriately determined according to the type of the tag. For example, referring to a combination of the above-mentioned tag and a substance capable of specifically binding to the tag, the substance can be selected from an antibody, a ligand receptor, an oligonucleotide, biotin, avidin (or streptavidin), a histidine tag or nickel, GST, glutathione, and the like. Among them, an antibody that specifically binds to the tag is preferred. The antibody may be a commercially available antibody or an antibody prepared by a method known in the art. The antibody may be a monoclonal antibody or a polyclonal antibody. The origin and isotype of the antibody are not particularly limited. Fragments of antibodies and derivatives thereof may be used, and examples thereof include Fab fragments, F(ab')2 fragments, and the like.

The method of this embodiment more preferably includes, between the forming a complex and the measuring of uptake amount, mixing a complex of the lipoprotein incorporating the labeled lipid and a first capture body with a second capture body that binds to the labeled lipid and forming a complex containing the lipoprotein incorporating the labeled lipid, the first capture body and the second capture body. In particular, it is preferable to form a complex of the lipoprotein incorporating the labeled lipid and a first capture body on a solid phase and then mix the complex immobilized on the solid phase with a second capture body. As a result, a complex of the first capture body, the lipoprotein incorporating the labeled lipid and the second capture body is formed. In this complex, the lipoprotein incorporating the labeled lipid is sandwiched between the first capture body and the second capture body. In this embodiment, the complex of the first capture body, the lipoprotein incorporating the labeled lipid and the second capture body is also referred to as a "sandwich complex".

The conditions of temperature and time in the contact between the labeled lipid and the second capture body are not particularly limited. For example, a mixture of a solution containing the complex of the lipoprotein and the first capture body and a solution containing the second capture body may be incubated at 4 to 60°C, preferably 25 to 42°C, for 1 second to 24 hours, preferably 1 minute to 2 hours. During the incubation, the mixture may be allowed to stand, or may be stirred or shaken.

(Second label)

The second capture body is preferably labeled with a second label. When the second capture body is labeled with a second label, the uptake amount can be measured by detecting a signal derived from the second label in the complex of the labeled lipid and the second capture body. The second label may be a signal generating substance, or a substance that catalyzes a reaction of other substances to generate a detectable signal can be used. Examples of the signal generating substance include fluorescent substances, radioactive isotopes, and the like. Examples of the substance that catalyzes the reaction of other substances to generate a detectable signal include enzymes. Examples of the enzyme include alkaline phosphatase, peroxidase, β-galactosidase, glucose oxidase, tyrosinase, acid phosphatase, luciferase, and the like. Examples of the fluorescent substances include fluorescent dyes such as fluorescein isothiocyanate (FITC), rhodamine and Alexa Fluor (registered trademark) and cyanine dyes, fluorescent proteins such as GFP, and the like.

Examples of the radioactive isotopes include $^{125}I$, $^{14}C$, $^{32}P$, and the like. Among them, an enzyme is preferable, and alkaline phosphatase and peroxidase are particularly preferable. Specific activity may be taken from these enzymes and used as a measured value of the uptake amount.

In this embodiment, labeling of the second capture body with the second label can be performed by directly or indirectly binding the second label to the second capture body. For example, the second label can be directly bonded to the second capture body using a commercially available labeling kit or the like. The second label may be indirectly bound to the second capture body by using a secondary antibody obtained by labeling an antibody capable of specifically binding to the second capture body with the second label. In this embodiment, a second capture body to which a second label is bound may be used, or a second capture body and a secondary antibody having a second label may be used.

In this embodiment, the washing of removing unreacted free components may be performed before signal detection. Examples of the unreacted free components include a free second capture body that did not bind to the tag, a free second label that did not bind to the second capture body, and the like. Specific washing method and washing solution are similar to those in the above-mentioned washing.

(Detection of signal derived from second label)

Methods for detecting a signal derived from the second label themselves are known in the art. In this embodiment, a suitable measurement method can be selected according to the type of signal derived from the second label. For example, when the second label is an enzyme, signals such as light and color generated by reacting an enzyme and a substrate for the enzyme can be measured by using a known apparatus. Examples of the measuring device include a spectrophotometer, a luminometer, and the like.

The substrate of the enzyme can be appropriately selected from known substrates according to the type of the enzyme. For example, when peroxidase is used as the enzyme, examples of the substrate include chemiluminescent substrates such as luminol and derivatives thereof, and chromogenic substrates such as 2,2'-azinobis(3-ethylbenzothiazoline-6-ammonium sulfonate) (ABTS), 1,2-phenylenediamine (OPD) and 3,3',5,5'-tetramethylbenzidine (TMB). When alkaline phosphatase is used as the enzyme, examples of the substrate include chemiluminescent substrates such as CDP-Star (registered trademark) (disodium 4-chloro-3-(methoxyspiro[1,2-dioxetane-3,2'-(5'-chloro)tricyclo[3.3.1.13,7]decan]-4-yl)phenyl phosphate) and CSPD (registered trademark) (disodium 3-(4-methoxyspiro[1,2-dioxetane-3,2-(5'-chloro)tricyclo[3.3.1.13,7]decan]-4-yl)phenyl phosphate), and chromogenic substrates such as 5-bromo-4-chloro-3-indolyl phosphate (BCIP), disodium 5-bromo-6-chloro-indolyl phosphate and p-nitrophenyl phosphate.

When the second label is a radioactive isotope, radiation as a signal can be measured using a known apparatus such as a scintillation counter. When the second label is a fluorescent substance, fluorescence as a signal can be measured using a known apparatus such as a fluorescence microplate reader. The excitation wavelength and the fluorescence wavelength can be appropriately determined according to the type of fluorescent substance used.

(Measurement of amount of lipoprotein immobilized on solid phase)

In this embodiment, the amount of lipoprotein immobilized on a solid phase (hereinafter, also referred to as "the amount of lipoprotein captured") may be measured, as necessary. This measurement may be performed separately from the measurement of uptake amount, or may be performed using a solid phase after the measurement of uptake amount. When the measurement is performed separately from the measurement of uptake amount, a plurality of solid phases on which lipoprotein incorporated with a labeled lipid are immobilized are prepared. Then, at least one solid phase is used to measure the amount of lipoprotein captured, and the remaining solid phase is used to measure the uptake amount.

The amount of lipoprotein captured itself can be measured based on a principle of known ELISA method. For example, it is possible to measure the amount of lipoprotein captured by contacting lipoprotein immobilized on a solid phase with a third capture body that binds to the lipoprotein to form a complex, and detecting the complex. The third capture body is not particularly limited as long as it is a substance capable of specifically binding to a part of the surface of lipoprotein. The details of the third capture body are similar to those described for the first capture body. The third capture body may be the same as or different from the first capture body. A preferred third capture body is an anti-ApoE antibody. When the first capture body and the third capture body are antibodies that bind to the same antigen (for example, ApoE), each other's epitopes are preferably different.

The third capture body is preferably labeled with a third label. When the third capture body is labeled with a third label, the amount of lipoprotein captured can be measured by detecting a signal derived from the third label in the complex of the lipoprotein immobilized on a solid phase and the third capture body. The details of the third label are similar to those described for the second label. The third label may be the same as or different from the second label. In this embodiment, the amount of lipoprotein captured can be measured by detecting the signal derived from the third label. The method of detecting the signal derived from the third label is similar to that described for the second label.

(Measured value of uptake amount in blood sample)

In this embodiment, the measurement result of the uptake amount can be obtained as information about cognitive function of the subject. Specifically, the measured value of the uptake amount in the blood sample of the subject is an indicator of cognitive impairment. The measured value of the uptake amount in the blood sample can be obtained as follows. For example, when the uptake amount is measured without diluting a predetermined amount of a blood sample (or a lipoprotein fraction prepared from a predetermined amount of a blood sample), the obtained measured value itself can be obtained as the measured value of the uptake amount in the blood sample. Alternatively, a value obtained by dividing the obtained measured value by a volume (value of a predetermined amount) of the blood sample used for measurement may be obtained as a measured value of the uptake amount per unit volume of the blood sample.

When the blood sample is diluted based on the concentration of apolipoprotein in the blood sample and used for measurement, the obtained measured value of the uptake amount may be converted to the measured value of the uptake amount per unit volume of the blood sample. This is because the concentration of lipoprotein in the blood sample is adjusted by dilution. For example, when the blood sample is diluted based on the concentration of apolipoprotein in the blood sample and used for measurement, the obtained measured value of the uptake amount can be converted to the measured value of the uptake amount per unit volume of the blood sample, by following equation (1).

(Measured value of uptake amount per unit volume of blood sample) = [(Measured value of uptake amount)/(Measured value of amount of lipoprotein captured)] × (Measured value of concentration of apolipoprotein in blood sample) ... (1) (1)

In the above equation (1), the measured value of the uptake amount per lipoprotein is obtained by dividing the measured value of the uptake amount by the measured value of the amount of lipoprotein captured. As mentioned above, the concentration of apolipoprotein in the blood sample is an indicator of the concentration of lipoprotein in the blood sample. Accordingly, the measured value of the uptake amount per unit volume of the blood sample is obtained by multiplying the measured value of the uptake amount per lipoprotein by the measured value of the concentration of apolipoprotein in the blood sample.

The measured value of the uptake amount in these blood samples and/or the measured value of the uptake amount per unit volume of the blood sample can be used as an indicator of cognitive impairment of this embodiment. That is, in this embodiment, a use of a measured value obtained by contacting lipoprotein in a blood sample of a subject with a labeled lipid and measuring the labeled lipid incorporated in the lipoprotein as an indicator of cognitive impairment is provided. The measured value of the uptake amount in the blood sample and/or the measured value of the uptake amount per unit volume of the blood sample is useful as an indicator for cognitive function of the subject.

The information about cognitive function of this embodiment may be, for example, information about risk of cognitive function decline of the subject and/or information indicating the state of cognitive function of the subject. More specifically, the information about cognitive function of this embodiment may include information indicating that a risk of declining cognitive function of the subject is high, information indicating that a risk of declining cognitive function of the subject is low, information indicating that cognitive function of the subject is declined, and information suggesting that cognitive function of the subject is not declined.

In this embodiment, when the measured value of the above-mentioned uptake amount is equal to or less than a predetermined threshold, the measured value suggests that a risk of declining cognitive function of the subject is high and/or that cognitive function of the subject is declined. When the measured value is greater than the predetermined threshold, the measured value suggests that the risk of declining the cognitive function of the subject is low and/or that the cognitive function of the subject is not declined.

In another embodiment, multiple thresholds are set. For example, it is possible to set a first threshold, and a second threshold greater than the first threshold, which classify the risk of cognitive function decline into three groups. According to this example, it is suggested that, when the measured value of the uptake amount is less than the first threshold, the risk of cognitive function decline is high, when the measured value is equal to or greater than the first threshold and less than the second threshold, the risk of cognitive function decline is moderate, and when the measured value is equal to or greater than the second threshold, the risk of cognitive function decline is low.

It is also possible to set a first threshold, and a second threshold that is greater than the first threshold, which classify the state of cognitive function of the subject into three groups. According to this example, it is suggested that, when the measured value of the uptake amount is less than the first threshold, the subject is AD, when the measured value is equal to or greater than the first threshold and less than the second threshold, the subject is MCI, and when the measured value is equal to or greater than the second threshold, the subject has normal cognitive function.

The predetermined threshold is not particularly limited. The predetermined threshold can be appropriately set. For example, a threshold for distinguishing subjects having normal cognitive function from subjects having abnormal

cognitive function may be set as follows. First, blood samples are obtained from a plurality of subjects (normal group) diagnosed with normal cognitive function and a plurality of subjects (abnormal group) diagnosed with abnormal cognitive function, and the uptake amount is measured to obtain measured value data. Then, a value capable of discriminating between the normal group and the abnormal group with the highest accuracy is obtained, and the value is set as a predetermined threshold. In setting the threshold, it is preferable to consider sensitivity, specificity, positive predictive value, negative predictive value, and the like.

The information about cognitive function of this embodiment may be, for example, information on onset risk of a central nervous system disease and/or information indicating the presence or absence of a central nervous system disease. More specifically, the information about cognitive function of this embodiment may include information indicating that the onset risk of a central nervous system disease of the subject is high, information indicating that the onset risk of a central nervous system disease of the subject is low, information indicating that the subject has a central nervous system disease, and information indicating that the subject does not have a central nervous system disease. The central nervous system disease of this embodiment is not particularly limited, and examples thereof include Alzheimer's disease, Parkinson's disease, and the like, and Alzheimer's disease is particularly preferable. Amyloid β, which is considered to be a causative substance of Alzheimer's disease, is produced by an action of β-secretase and γ-secretase. It is known that activities of β-secretase and γ-secretase decrease as the cholesterol density decreases, and it is considered that when the uptake amount is large, the β-secretase and γ-secretase activities decrease and amyloid β production is suppressed. Therefore, it is considered that the greater the uptake amount, the lower the onset risk of Alzheimer's disease. Accordingly, the method of this embodiment can be suitably used as a means for obtaining information on Alzheimer's disease.

In this embodiment, when the measured value of the above-mentioned uptake amount is equal to or less than a predetermined threshold, the measured value suggests that the risk of developing a central nervous system disease is high and/or that the subject develops a central nervous system disease. When the measured value is greater than the predetermined threshold, the measured value suggests that the risk of developing a central nervous system disease is low and/or that the subject does not develop a central nervous system disease.

[2. Method for Determining Effectiveness of Medical Intervention about Cognitive Function]

The measurement result of the uptake amount obtained by the method of this embodiment can be used to determine effectiveness of medical intervention about cognitive function. In the method for determining effectiveness of medical intervention about cognitive function of a subject of this embodiment, first, before medical intervention about cognitive function on a subject and after performing the medical intervention, lipoprotein in a blood sample of the subject is contacted with a labeled lipid, and the labeled lipid incorporated into the lipoprotein is measured. This measurement is the same as described above for the method for obtaining information about cognitive function. Subsequently, the measured value obtained by the measurement before the medical intervention is compared with the measured value obtained by the measurement after the medical intervention to determine effectiveness of the medical intervention. When the measured value obtained by the measurement after the medical intervention is greater than the measured value obtained by the measurement before the medical intervention, it can be determined that the medical intervention is effective for the subject. When the measured value obtained by the measurement after the medical intervention is equal to or less than the measured value obtained by the measurement before the medical intervention, it can be determined that the medical intervention is not effective for the subject.

The medical intervention includes administration of therapeutic and prophylactic agents, diet therapy, exercise therapy, learning therapy, surgery, immunotherapy, gene therapy, and the like.

In the above determination, when the measured value obtained by the measurement after the medical intervention was the same as the measured value obtained by the measurement before the medical intervention, it was determined that the medical intervention was not effective for the subject. However, the medical intervention may be determined to be effective for the subject. That is, when the measured value obtained by the measurement after the medical intervention is equal to or greater than the measured value obtained by the measurement before the medical intervention, it can be determined that the medical intervention is effective for the subject. When the measured value obtained by the measurement after the medical intervention is lower than the measured value obtained by the measurement before the medical intervention, it can be determined that the medical intervention is not effective for the subject. When the measured value obtained by the measurement after the medical intervention is the same as the measured value obtained by the measurement before the medical intervention, it can be determined that follow-up is required.

The information about the cognitive function of the subject of this embodiment may be obtained by continuously monitoring the subject. In the method of monitoring (hereinafter referred to as the monitoring method), for example, using the method of this embodiment described above, an uptake amount in a first blood sample obtained from a subject of a first intervention and an uptake amount in a second blood sample obtained from the subject of a second intervention are

measured. In the monitoring method of this embodiment, effectiveness of medical interventions about cognitive function can be monitored by comparing the measured values of the first and second interventions.

A time point of the first intervention is not particularly limited and can be any time point. A time point of the second intervention is not particularly limited as long as it is different from the time point of the first intervention. The second intervention may be a time point when, for example, a period selected from the range of 1 day to 1 year has passed from the first intervention. Specifically, a period between the first intervention and the second intervention can be about 3 months. For example, a blood sample can be taken from a subject and measured about every 3 months. The first and second interventions may be the same medical intervention or different medical interventions. The measurements of the first and second blood samples may be performed substantially simultaneously or sequentially. When the first and second blood samples are measured substantially simultaneously, it is preferable to properly store the first blood sample until the measurement. If necessary, the second blood sample may also be properly stored until the measurement.

When the measured value at the time point of the second intervention is equal to or greater than the measured value at the time point of the first intervention, it can be determined that the medical intervention is effective for the subject. When the measured value at the time point of the second intervention is less than the measured value at the time point of the first intervention, it can be determined that the medical intervention is not effective for the subject. From the measured value at the time point of the second intervention, the health care professional may determine that the subject needs to continue monitoring.

[3. Method for Assisting Determination of Cognitive Function of Subject]

The measured value of the uptake amount can be used to assist determination of cognitive function of a subject. In the method for assisting determination of cognitive function of a subject of this embodiment, first, lipoprotein in a blood sample of a subject is contacted with a labeled lipid, and the labeled lipid incorporated into the lipoprotein is measured. This measurement is the same as described above for the method for obtaining information about cognitive function. Subsequently, when the measured value obtained by the measurement is equal to or less than a predetermined threshold, it is determined that a risk of declining cognitive function of the subject is high, and/or it can be determined that cognitive function of the subject is declined. When the measured value obtained by the measurement is greater than the predetermined threshold, it is determined that the risk of declining cognitive function of the subject is low, and/or it can be determined that the cognitive function of the subject is not declined.

In the above determination, when the measured value obtained by the measurement is same as the predetermined threshold, it was determined that the risk of declining cognitive function of the subject was high, but it may be determined that the risk of declining cognitive function of the subject is low. That is, when the measured value obtained by the measurement is less than the predetermined threshold, it is determined that a risk of declining cognitive function of the subject is high, and/or it can be determined that cognitive function of the subject is declined. In the determination, when the measured value obtained by the measurement is equal to or greater than the predetermined threshold, it is determined that the risk of declining cognitive function of the subject is low, and/or it can be determined that the cognitive function of the subject is not declined.

With reference to findings obtained by the method for assisting determination, it may be predicted that a subject determined to have a high risk of declining cognitive function by a health care professional such as a doctor may be predicted to have, for example, a high possibility of developing a central nervous system disease within 5 years, without medical intervention. On the other hand, it may be predicted that a subject determined to have a low risk of declining cognitive function by a health care professional such as a doctor may be predicted to have, for example, a low possibility of developing a central nervous system disease within 5 years. A subject who is determined to have declined cognitive function by a health care professional such as a doctor may be determined to require medical intervention. A subject who is determined not to have declined cognitive function by a health care professional such as a doctor may be determined not to require medical intervention. Thus, the method for assisting determination of cognitive function of this embodiment makes it possible to provide a health care professional such as a doctor with information that assists in determining a risk of declining cognitive function and/or cognitive function. By actively providing medical intervention such as medication to suppress onset and symptoms of central nervous system diseases to a subject determined to have a high risk of declining cognitive function by the method for assisting determination of cognitive function of this embodiment, it is possible to prevent the onset of central nervous system diseases and alleviate the symptoms.

In another embodiment, multiple thresholds are set. For example, it is possible to set a first threshold, and a second threshold greater than the first threshold, which classify the risk of declining cognitive function into three groups. According to this example, it is suggested that, when the measured value of the uptake amount is less than the first threshold, the risk of declining cognitive function is high, when the measured value is equal to or greater than the first threshold and less than the second threshold, the risk of declining cognitive function is moderate, and when the measured value is equal to or greater than the second threshold, the risk of declining cognitive function is low.

It is also possible to set a first threshold, and a second threshold that is greater than the first threshold, which

classify the state of cognitive function of the subject into three groups. According to this example, when the measured value of the uptake amount is less than the first threshold, it is determined that the cognitive function of the subject is declined, when the measured value is equal to or greater than the first threshold and less than the second threshold, it is determined that follow-up is required, and when the measured value is equal to or greater than the second threshold, it is determined that the cognitive function is not declined. In these examples, the first threshold is a value lower than the second threshold.

When it is determined that follow-up is required, the risk of declining cognitive function is not low, but it does not require medical intervention such as medication. That is, a subject is classified as having a medium risk of declining cognitive function. Accordingly, it can be predicted that the subject needs to continue an examination for cognitive function.

In the above determination, when the measured value of the uptake amount in the blood sample is the same as the first threshold, it was determined that the risk of declining cognitive function was high, but it may be determined that follow-up is required. Further, in the above determination, when the measured value of the uptake amount in the blood sample is the same as the second threshold, it was determined that the risk of declining cognitive function was low, but it may be determined that follow-up is required.

A health care professional such as a doctor may use the measured value as an indicator for determining cognitive function, or may determine the state of cognitive function by combining the measured value with other information. The term "other information" includes, for example, information obtained by Mini-Mental State Examination (MMSE), measurement of amyloid β40 or amyloid β42 in cerebrospinal fluid (CSF) or the blood sample, diagnostic imaging of amyloid β by PET, diagnostic imaging of tau protein by PET, measurement of tau protein (total tau, phosphorylated tau) in blood or CSF, and the like, and other medical findings.

[4. Reagent Kit for Obtaining Information about Cognitive Function]

The scope of the present disclosure also includes a reagent kit for obtaining information about cognitive function. That is, a reagent kit for obtaining information about cognitive function (hereinafter, also referred to as a "reagent kit"), including a labeled lipid and a capture body that binds to lipoprotein is provided. The details of the labeled lipid and the capture body that binds to lipoprotein are the same as described for the labeled lipid and the first capture body used in the method of this embodiment. The reagent kit of this embodiment includes one or more reagents.

When the labeled lipid is tagged sterol, the reagent kit of this embodiment may further include a capture body that binds to the tag. The reagent kit of this embodiment may further include a labeling substance for labeling the capture body. In this embodiment, the labeling substance may be previously bound to the capture body (the obtained capture body is also referred to as the "labeled capture body"). Thus, the reagent kit of this embodiment may further include a labeled capture body that binds to the tag. When the labeling substance is an enzyme, the reagent kit of this embodiment may further contain a substrate for the enzyme. The details of the capture body that binds to the tag, the labeling substance and the substrate are the same as those described for the second capture body, the second label and the detection of the signal derived from the label.

The reagent kit of this embodiment may include a capture body for measuring the amount of lipoprotein captured. The capture body is not particularly limited as long as it is a substance capable of specifically binding to a part of the surface of lipoprotein. The details of the capture body are similar to those described for the third capture body. The capture body for measurement of the amount of lipoprotein captured may be the same as or different from the capture body that binds to lipoprotein. When these capture bodies are antibodies that bind to the same antigen (for example, ApoE), each other's epitopes are preferably different.

The reagent kit of this embodiment may further include a labeling substance for labeling a capture body for measuring the amount of lipoprotein captured. Alternatively, the reagent kit of this embodiment may further include a labeled capture body for measuring the amount of lipoprotein captured. When the labeling substance is an enzyme, the reagent kit of this embodiment may further contain a substrate for the enzyme. The details of the labeling substance and the substrate are the same as those described for the third label and the detection of the signal derived from the label.

The reagent kit of this embodiment may further contain a first additive and/or a second additive as a stabilizing reagent that reduces variation of measured values of the uptake amount. Details of the first additive and/or the second additive are as mentioned above. As used herein, the term "stabilizing reagent" refers to a reagent that improves reproducibility of measurement.

The reagent kit of this embodiment may include a reagent containing both a labeled lipid and a first additive and/or a second additive. Alternatively, the measurement reagent of this embodiment may be a two-reagent form including a first reagent containing a labeled lipid and a second reagent containing a first additive and/or a second additive.

The reagent kit of this embodiment may further include a substance useful in measuring the uptake amount. Examples of the substance useful for measurement include surfactants having no cyclic structure, cyclic oligosaccharides, components that bind to a lipoprotein different from the lipoprotein to be measured, blocking agents, and the like. Details of these substances are as mentioned above.

It is preferable that the labeled lipid, the various capture bodies, the stabilizing reagent, the labeling substance

and the substrate be stored in separate containers or individually packaged. The forms of the labeled lipid, the various capture bodies, the stabilizing reagent, the labeling substance and the substrate are not particularly limited, and they may be a solid (for example, powder, crystal, freeze-dried product, and the like) or liquid (for example, solution, suspension, emulsion, and the like). In the case of a liquid, examples of the solvent include water, physiological saline, PBS, Tris-HCl, Good buffer, and the like, which may further contain a substance useful in measuring the uptake amount. These solvents may be separately stored in a container as a sample dilution reagent.

The reagent kit of this embodiment may be provided to a user by packing a container containing a first reagent and a container containing a second reagent in a box. The box may contain a package insert describing how to use the reagents and the like. The attached document may describe a configuration of the reagent kit, method of use, relationship between the value measured by the reagent kit and the state of cognitive function, and the like. Examples of the reagent kit are shown in some figures below. However, this embodiment is not limited to these examples. For example, Fig. 1A shows another example of the reagent kit of this embodiment. In Fig. 1A, 10 denotes a reagent kit, 11 denotes a first container containing a labeled lipid, 12 denotes a second container containing a capture body that binds to lipoprotein, 13 denotes a packing box, and 14 denotes an attached document.

Fig. 1B shows another example of the reagent kit of this embodiment. In Fig. 1B, 20 denotes a reagent kit, 21 denotes a first container containing a labeled lipid, 22 denotes a second container containing a sample dilution reagent, 23 denotes a third container containing a liquid reagent containing a first additive and/or a second additive, 24 denotes a fourth container containing a first capture body, 25 denotes a fifth container containing a second capture body, 26 denotes a sixth container containing a suspension containing a particle as a solid phase, 27 denotes a packing box, and 28 denotes a package insert.

The reagent kit of this embodiment may include a microplate instead of the container containing a suspension containing a particle. The reagent kit of this embodiment may include a container in which a first capture body is immobilized on a particle in advance and a suspension containing the particle to which the first capture body is immobilized is stored. When the second label is an enzyme, the reagent kit of this embodiment may further include a substrate for the enzyme and a buffer necessary for an enzyme reaction. Fig. 2 shows another example of the reagent kit of this embodiment. In Fig. 2, 30 denotes a reagent kit, 31 denotes a first container containing a labeled lipid, 32 denotes a second container containing a capture body that binds to lipoprotein, 33 denotes a packing box, 34 denotes an attached document, and 35 denotes a 96-well microplate as a solid phase.

The reagent kit of this embodiment may include a reagent for measuring an indicator for other cognitive functions such as amyloid β, in addition to the above-mentioned reagent for measuring the uptake amount. If such a reagent is, for example, a reagent for measuring amyloid β, the reagent kit of this embodiment may also include a 96-well plate pre-coated with an anti-amyloid β antibody or the like. In this case, for example, the same blood sample may be divided and the uptake amount and the amyloid β concentration may be quantified respectively.

[5. Determination Apparatus and Computer Program]

The scope of the present disclosure also includes a device and a computer program for implementing the method of this embodiment. An example of the apparatus for determining a risk of cognitive function decline of this embodiment will be described with reference to the drawings. However, this embodiment is not limited only to the embodiment shown in this example. A determination apparatus 100 shown in Fig. 3 includes a measuring device 200 and a computer system 300 connected to the measuring device 200.

The measuring device is not particularly limited as long as it can detect a signal based on a labeled lipid incorporated into lipoprotein. The measuring device can be appropriately selected according to the type of label of the labeled lipid. In the above example, the measuring device 200 is a plate reader that detects a signal based on the labeled lipid incorporated into the lipoprotein on the microplate. The signal is optical information such as a fluorescence signal. In this case, when a microplate on which a complex containing the lipoprotein incorporating the labeled lipid is immobilized is set in the measuring device 200, the measuring device 200 obtains optical information based on the labeled lipid, and the measuring device 200 transmits the obtained optical information to the computer system 300.

As necessary, the amount of lipoprotein captured may be measured by the measuring device. In this case, when the microplate on which a complex of lipoprotein and a labeled capture body that binds to the lipoprotein is immobilized is set in the measuring device 200, the measuring device 200 obtains optical information based on the labeled capture body, and the measuring device 200 transmits the obtained optical information to the computer system 300. The microplate for measuring the amount of lipoprotein captured and the microplate for measuring the uptake amount may be the same or different. The label capture body is the same as the third capture body labeled with the third label.

The computer system 300 includes a computer main body 301, an input unit 302, and a display unit 303 that displays specimen information, a determination result, and the like. The computer system 300 receives the optical information from the measuring device 200. Then, the processor of the computer system 300 executes a program for determining a risk of cognitive function decline, based on the optical information. As shown in Fig. 3, the computer system

300 may be equipment separate from the measuring device 200, or may be equipment including the measuring device 200. In the latter case, the computer system 300 may itself be the determination apparatus 100.

With reference to Fig. 4, the computer main body 301 includes a central processing unit (CPU) 310, a read only memory (ROM) 311, a random access memory (RAM) 312, a hard disk 313, an input/output interface 314, a reading device 315, a communication interface 316, and an image output interface 317. The CPU 310, the ROM 311, the RAM 312, the hard disk 313, the input/output interface 314, the reading device 315, the communication interface 316 and the image output interface 317 are data-communicably connected by a bus 318. The measuring device 200 is communicably connected to the computer system 300 via the communication interface 316.

The CPU 310 can execute a program stored in the ROM 311 or the hard disk 313 and a program loaded in the RAM 312. The CPU 310 obtains a measured value of the uptake amount in the blood sample based on the optical information obtained from the measuring device 200. Details of the measured value of the uptake amount in the blood sample and the calculation thereof are the same as those described for the method of this embodiment. When the diluted blood sample (or lipoprotein fraction) is used, the measured value of the uptake amount in the blood sample is calculated according to the equation (1) stored in ROM 311 or the hard disk 313. Then, the CPU 310 determines cognitive function based on the obtained measured value and the predetermined threshold stored in the ROM 311 or the hard disk 313. The CPU 310 outputs the determination result. The CPU 310 displays the determination result on the display unit 303.

The ROM 311 includes a mask ROM, PROM, EPROM, EEPROM, and the like. In the ROM 311, a computer program executed by the CPU 310 and data used for executing the computer program are recorded. The computer program recorded in ROM 311 includes a basic input output system (BIOS).

The RAM 312 includes SRAM, DRAM, and the like. The RAM 312 is used for reading the program recorded in the ROM 311 and the hard disk 313. The RAM 312 is also used as a work area of the CPU 310 when these programs are executed.

The hard disk 313 has installed therein an operating system to be executed by the CPU 310, a computer program such as an application program (program for determining cognitive function), and data used for executing the computer program.

The reading device 315 includes a flexible disk drive, a CD-ROM drive, a DVD-ROM drive, a USB port, an SD card reader, a CF card reader, a memory stick reader, a solid state drive, and the like. The reading device 315 can read a program or data recorded on a portable recording medium 400.

The input/output interface 314 includes, for example, a serial interface such as USB, IEEE1394 and RS-232C, a parallel interface such as SCSI, IDE and IEEE1284, and an analog interface including a D/A converter, an A/D converter and the like. The input unit 301 such as a keyboard and a mouse is connected to the input/output interface 314. An operator can input various commands to the computer main body 301 through the input unit 302.

The communication interface 316 is, for example, an Ethernet (registered trademark) interface or the like. The computer main body 301 can also transmit print data to a printer or the like through the communication interface 316.

The image output interface 317 is connected to the display unit 302 including an LCD, a CRT, and the like. As a result, the display unit 303 can output a video signal corresponding to the image data coming from the CPU 310. The display unit 303 displays an image (screen) according to the input video signal.

Referring to Fig. 5A, a processing procedure for determining a risk of declining cognitive function executed by the determination apparatus 100 will be described. Here, a case of determining a risk of declining cognitive function based on a fluorescence signal generated from a fluorescently labeled lipid incorporated into lipoprotein on the microplate will be described as an example. However, this embodiment is not limited to this example.

The CPU 310 obtains optical information (fluorescent signal) from the measuring device 200 in step S101. The CPU 310 obtains a measured value of a labeled lipid incorporated into lipoprotein from the obtained optical information in step S102. The measured value is stored in the hard disk 313 (may be stored in the RAM 312. Here, the case of storing in the hard disk 313 will be described). In step S103, the CPU 310 compares the measured value with the predetermined threshold stored in the hard disk 313. When the measured value is less than the predetermined threshold, the process proceeds to step S104, and a determination result indicating that the risk of declining cognitive function is high is stored in the hard disk 313. When the measured value is equal to or greater than the predetermined threshold, the process proceeds to step S105, and the determination result indicating that the risk of declining cognitive function is low is stored in the hard disk 313 (may be stored in RAM 312). In step S106, the CPU 310 outputs the determination result. Specifically, the determination result is displayed on the display unit 303 or printed by a printer. Accordingly, it is possible to provide doctors and the like with information that assists in determining the risk of declining cognitive function.

Referring to Fig. 5B, a processing procedure for determining a possibility that cognitive function is declined executed by the determination apparatus 100 will be described. Here, a case of determining a possibility that cognitive function is declined based on a fluorescence signal generated from a fluorescently labeled lipid incorporated into lipoprotein on the microplate will be described as an example. However, this embodiment is not limited to this example.

Details of steps S201, S202 and S206 are similar to those described for the steps S101, S102 and S106, respectively. Here, a case where the measured value is stored in the hard disk 313 will be described. In step S203, the CPU

310 compares the calculated measured value with the predetermined threshold stored in the hard disk 313. When the measured value is less than the predetermined threshold, the process proceeds to step S204, and a determination result indicating that the possibility that cognitive function is declined is high is stored in the hard disk 313. When the measured value is equal to or greater than the predetermined threshold, the process proceeds to step S205, and the determination result indicating that the possibility that cognitive function is declined is low is stored in the hard disk 313 (may be stored in RAM 312). Accordingly, it is possible to provide doctors and the like with information that assists in determining the possibility that cognitive function is declined.

Referring to Fig. 5C, a processing procedure for determining the risk of declining cognitive function and the possibility that cognitive function is declined executed by the determination apparatus 100 will be described. Here, a case of determining the risk of declining cognitive function and the possibility that cognitive function is declined based on a fluorescence signal generated from a fluorescently labeled lipid incorporated into lipoprotein on the microplate will be described as an example. However, this embodiment is not limited to this example.

Details of steps S301, S302 and S306 are similar to those described for the steps S101, S102 and S106, respectively. Here, a case where the measured value is stored in the hard disk 313 will be described. In step S303, the CPU 310 compares the calculated measured value with the predetermined threshold stored in the hard disk 313. When the measured value is less than the predetermined threshold, the process proceeds to step S304, and a determination result indicating that the risk of declining cognitive function and the possibility that cognitive function is declined are high is stored in the hard disk 313. When the measured value is equal to or greater than the predetermined threshold, the process proceeds to step S305, and the determination result indicating that the risk of declining cognitive function and the possibility that cognitive function is declined are low is stored in the hard disk 313 (may be stored in RAM 312). Accordingly, it is possible to provide doctors and the like with information that assists in determining the risk of declining cognitive function and the possibility that cognitive function is declined.

Referring to Fig. 6, a processing procedure for determining a risk of declining cognitive function using two predetermined thresholds will be described. Here, a case of determining a risk of declining cognitive function based on a fluorescence signal generated from a fluorescently labeled lipid incorporated into lipoprotein on the microplate will be described as an example. However, this embodiment is not limited to this example.

Details of steps S401, S402 and S408 are similar to those described for the steps S101, S102 and S106, respectively. Here, a case where the measured value is stored in the hard disk 313 will be described. In step S403, the CPU 310 compares the calculated measured value with the first threshold stored in the hard disk 313. When the measured value is less than the first threshold, the process proceeds to step S404, and a determination result indicating that the risk of declining cognitive function of the subject is high is stored in the hard disk 313. When the measured value is equal to or greater than the first threshold, the process proceeds to step S405. In step S405, the CPU 310 compares the calculated measured value with the second threshold stored in the hard disk 313. When the measured value is equal to or greater than the second threshold, the process proceeds to step S406, and a determination result indicating that the risk of declining cognitive function of the subject is low is stored in the hard disk 313. When the measured value is less than the second threshold, the process proceeds to step S407, and a determination result indicating that the subject requires follow-up is stored in the hard disk 313 (may be stored in RAM 312).

Hereinafter, the present disclosure will be described in more detail by examples, but the present disclosure is not limited to these examples.

## EXAMPLES

### Example 1: Construction of measurement system for uptake amount

Using labeled lipids and anti-ApoE antibodies, a measurement system was constructed to measure an uptake amount.

(1) Measurement of uptake amount using anti-ApoE antibody

(1.1) Preparation of measurement plate (Immobilization of anti-ApoE antibody to solid phase)

A commercially available anti-ApoE antibody (Biolegend) was used as the anti-apolipoprotein antibody. To each well of a 96-well microplate (black plate H for fluorescence measurement, Sumitomo Bakelite Co., Ltd.) as a solid phase was added 200 μl each of 50 mM Tris-HCl (pH 7.5) for washing. This washing operation was performed twice in total. To each well were added 100 μl each of 50 mM Tris-HCl (pH 7.5) and 5 μg/ml anti-ApoE antibody solution, and the mixture was allowed to stand at 4°C overnight or longer. The antibody solution was removed, and 200 μl each of PBS was added to each well for washing. This washing operation was performed three times in total. To each well was added 200 μl each of StartingBlock (registered trademark) (PBS) Blocking Buffer (Thermo Scientific), and the mixture was shaken at 600 rpm at

25°C for 2 hours.

(1.2) Formation and measurement of complex of anti-ApoE antibody and lipoprotein on solid phase

(i) Preparation of measurement sample (Contact between lipoprotein and tagged cholesterol)

As a blood sample, blood was taken from a healthy person and serum was prepared. The ApoE concentration of this serum was measured using an ApoE measurement kit (NITTOBO). Specific operations for measuring the concentration were performed according to a manual attached to the kit. After the measurement, each specimen was diluted with dilution buffer 1 (PBS containing 1% BSA) to prepare lipoprotein-containing diluents having ApoE concentration of 0.28, 0.57, 1.13, 2.8 and 5.7 μg/ml. Dilution buffer 1 was used as a control specimen (ApoE concentration 0 μg/ml) containing no lipoprotein fraction. PBS was prepared by dissolving Phosphate buffered saline tablet (Sigma-Aldrich Co. LLC.) in water.

To a reaction buffer (1.1% Pluronic F68 (Thermo Scientific), 0.11 mM methyl-β-cyclodextrin, 0.0011% liposome, 0.0033% nonion K-230, 0.366% LIPIDURE (registered trademark) SF-08) was added 0.1 mM Biotin-PEG7-added cholesterol (see Examples 2 and 4 of US2017/0315112 for the method of preparing Biotin-PEG7-added cholesterol) to a final concentration of 3.3 μM, then the above lipoprotein-containing diluent was further added thereto in an amount of 1/10 of the total amount. The obtained mixture was shaken at room temperature at 1,000 rpm for 30 minutes to prepare a measurement sample. The composition of the liposome contained in the reaction buffer is 139 nM dimyristoyl phosphatidyl glycerol (DMPG), 139 nM cholesterol and 278 nM hydrogenated soybean phosphatidyl choline (HSPC).

(ii) Formation of complex of lipoprotein incorporating cholesterol and anti-ApoE antibody

Blocking buffer (StartingBlock, Thermo Scientific) was removed from the plate on which the anti-ApoE antibody was immobilized, and 100 μl each of measurement samples was added to wells. Then, the plate was shaken at 600 rpm at room temperature for 1 hour to form a complex of the lipoprotein and the anti-ApoE antibody.

(iii) Measurement of amount of cholesterol incorporated into lipoprotein

The plate prepared in (ii) above was washed 3 times with a washing solution (HISCL Line Washing Solution (Sysmex Corporation), 138 mM NaCl, 0.1% Pluronic F68), 100 μl each of Streptavidin, Alkaline Phosphatase conjugated (Vector)/Dilution buffer 2 (0.1 M TEA, 10 mg/mL BSA, 5 mg/mL sodium caseinate, 1 mM $MgCl_2$, 0.1 mM $MnCl_2$) were added to each well, and the plate was shaken at 600 rpm at room temperature for 1 hour. Then, the plate was washed 5 times with a washing solution, 100 μl each of a luminescent substrate (a 1:2 mixture of HISCL R4 reagent and HISCL R5 reagent (Sysmex Corporation)) was added to the wells. The plate was shaken for 30 minutes. Chemiluminescence intensity was measured with (Infinite (registered trademark) 200 Pro, TECAN) with a plate reader.

(iv) Measurement of amount of captured lipoprotein

The plate prepared in (ii) above was washed 3 times with a washing solution, goat anti-ApoE serum of the ApoE measurement kit was diluted 3,000 times with a blocking buffer (StartingBlock, Thermo Scientific), and 100 μl each of the obtained diluent was added to each well. The plate was shaken at 600 rpm at 25°C for 1 hour, then the diluent was removed and each well was washed three times with a washing solution. HRP-labeled rabbit anti-goat IgG polyclonal antibody (P0449, Dako) was diluted 3,000-fold with a blocking buffer (StartingBlock, Thermo Scientific), and 100 μl each of the obtained diluent was added to each well. The plate was shaken at 600 rpm at 25°C for 1 hour, then the diluent was removed and each well was washed five times with a washing solution. 100 μl each of a chemiluminescent substrate solution (Super Signal ELISA Pico, 37069, Thermo Scientific) was added to each well. After shaking the plate at 600 rpm at 25°C for 2 minutes, the amount of luminescence was measured with a microplate reader (Infinite (registered trademark) F200 Pro, TECAN).

(1.3) Measurement results

From the measurement results of (iv) above, it was found that the amount of lipoprotein prepared in (i) above could be captured on the plate according to the ApoE concentration (not shown). That is, a linear correlation was observed between the ApoE concentration in a diluent containing a lipoprotein fraction and the amount of lipoprotein captured measured in (iv) above. Fig. 7 shows the relationship between the ApoE concentration in the diluent containing a lipoprotein fraction and the measurement result of the amount of cholesterol uptake by lipoprotein measured in (iii) above. As can be seen from Fig. 7, a high correlation was observed between the ApoE concentration in the specimen (the amount of lipoprotein captured by the anti-ApoE antibody) and the amount of cholesterol incorporated into the lipoprotein.

(2) Evaluation of activity between ApoE genotypes

As mentioned above, it is known that an ability to extract cholesterol from cells decreases in the order of ApoE2 > ApoE3 > ApoE4 depending on the ApoE genotype. Whether or not such a difference could be evaluated was verified by measuring the uptake amount using recombinant ApoE protein and anti-ApoE.

(2.1) Preparation of recombinants ApoE

Three recombinant proteins, ApoE2, ApoE3 and ApoE4 (all purchased from FUJIFILM Wako Pure Chemical Corporation) were used. Recombinants ApoE2, ApoE3 and ApoE4 were each modified with 6 M guanidine hydrochloride and 1 mM dithiothreitol (DTT) and then dialyzed against PBS containing 0.5 mM DTT. The ApoE concentration after refolding was adjusted to 1 μg/ml, and diluents containing each recombinant ApoE were obtained. As a control, a diluent to which recombinant ApoE was not added was used. Each of the obtained recombinant ApoE diluents was measured in the same manner as in (1.2) above.

(2.2) Measurement result

Fig. 8 shows the measurement result of the uptake amount. In the figure, "none" indicates a diluent to which recombinant ApoE was not added. As shown in Fig. 8, the uptake amount was highest when the recombinant ApoE2 was added. When recombinant ApoE3 was added, the uptake amount was higher than when recombinant ApoE4 was added. This result is consistent with the report of J Neurochem. 2000 Mar: 74 (3): 1008-16. From this, it was shown that the difference in the genotype of ApoE can be evaluated by measuring the uptake amount of labeled lipid by lipoprotein using the anti-ApoE antibody of this embodiment. The ApoE genotype is known to be a risk factor for cognitive function. Accordingly, it was shown that the measurement method of this embodiment, which correlates with the ApoE genotype, can be used as a

| | Normal cognitive function (CH) | Mild cognitive impairment (MCI) | Alzheimer's disease (AD) |
|---|---|---|---|
| Number of subjects | 8 | 8 | 8 |
| Age | 67±10 | 69±17 | 70±16 |
| Sex (Proportion of male) | 50 | 50 | 50 |
| MMSE Point | 30±0 | 24±2 | 17±4 |

measurement method of risk factors.

Example 2: Evaluation of clinical specimens

In Example 2, whether or not information about cognitive function could be obtained was verified using a specimen derived from a patient.

(1) Subject information

Blood and CSF were collected from 8 subjects with normal cognitive function (Cognitively Health: CH), 8 subjects with mild cognitive impairment (MCI) and 8 subjects with Alzheimer's disease (AD), and used as biological samples. Information on each subject group was as shown in Table 1 below.
[Table 1]

(2) Functional evaluation of ApoE lipoprotein

Serum was prepared from the blood obtained from the subject of (1) above, and a correlation between the uptake amount and cognitive impairment was verified.

Measurement results

The serum obtained from the subject was measured in the same manner as in (1.2) above, and the measurement result of the obtained uptake amount is shown in Fig. 9. Fig. 9 shows degrees of uptake amounts of MCI group and AD

group, based on 100% of the uptake amount of CH group. As shown in Fig. 9, a significant difference was observed in the uptake amount between the CH group and the MCI group, and between the CH group and the AD group. On the other hand, the amount of ApoE lipoprotein captured when serum obtained from the same subject was measured in the same manner as in (1.2) (iv) above is shown in Fig. 10 (the amounts of the MCI group and the AD group captured are shown, based on 100% of the captured amount of the CH group). As shown in Fig. 10, a significant difference was not observed in the captured amount between the CH group and the MCI group, and between the CH group and the AD group. Based on the above, it was suggested that information about cognitive function (particularly cognitive function due to Alzheimer's disease) can be obtained by measuring the uptake amount in a blood sample by the uptake amount measurement method using the anti-ApoE antibody of this embodiment.

## Claims

**1.** A method for obtaining information on cognitive function of a subject, comprising: contacting lipoprotein in a blood sample of a subject with a labeled lipid; and measuring the labeled lipid incorporated into the lipoprotein to obtain a measured value, wherein the measured value is an indicator of cognitive impairment;

wherein the labeled lipid is a labeled cholesterol and the lipoprotein comprises ApoE,
wherein the contacting comprises: contacting the lipoprotein incorporating the labeled lipid with a capture body that binds to the lipoprotein to form a complex, wherein the complex comprises the lipoprotein incorporating the labeled lipid and the capture body, and
wherein the capture body is an anti-ApoE antibody.

**2.** The method according to claim 1, wherein the information on cognitive function is information on risk of cognitive function decline of the subject and/or information indicating a state of cognitive function of the subject.

**3.** The method according to claim 2, wherein, when the measured value is equal to or less than a predetermined threshold, the measured value suggests that a risk of declining the cognitive function of the subject is high and/or that the cognitive function of the subject is declined.

**4.** The method according to claim 2, wherein, when the measured value is greater than a predetermined threshold, the measured value suggests that a risk of declining the cognitive function of the subject is low and/or that the cognitive function of the subject is not declined.

**5.** The method according to claim 1, wherein the information on cognitive function is information on onset risk of a central nervous system disease and/or information indicating a presence or absence of a central nervous system disease.

**6.** The method according to claim 5, wherein, when the measured value is equal to or less than a predetermined threshold, the measured value suggests that the risk of developing a central nervous system disease is high and/or that the subject develops a central nervous system disease.

**7.** The method according to claim 5, wherein, when the measured value is greater than a predetermined threshold, the measured value suggests that the risk of developing a central nervous system disease is low and/or that the subject does not develop a central nervous system disease.

**8.** The method according to any one of claims 5 to 7, wherein the central nervous system disease is Alzheimer's disease.

**9.** The method according to claim 1, wherein

the labeled sterol is tagged cholesterol represented by a following formula (I):

[Chemical Formula 1]

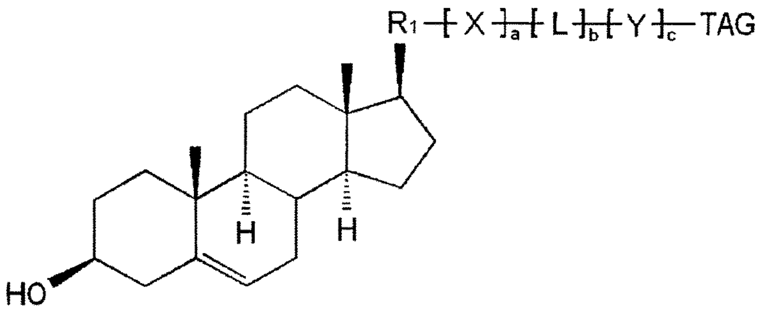

(I)

wherein $R_1$ is an alkylene group having 1 to 6 carbon atoms which optionally have a methyl group;
X and Y are identical or different, and are represented by $-R_2$-NH-, -NH-$R_2$-, $-R_2$-(C=O)-NH-, -(C=O)-NH-$R_2$-, $-R_2$-NH-(C=O)-, -NH-(C=O)-$R_2$-, $-R_2$-(C=O)-, -(C=O)-$R_2$-, $-R_2$-(C=O)-O-, - (C=O)-O-$R_2$-, $-R_2$-O-(C=O)-, -O-(C=O)-$R_2$-, $-R_2$-(C=S)-NH-, - (C=S)-NH-$R_2$-, $-R_2$-NH-(C=S)-, -NH- (C=S)-$R_2$-, $-R_2$-O-, -O-$R_2$-, - $R_2$-S-, or -S-$R_2$-, and each $R_2$ is independently an atomic bonding, an alkylene group having 1 to 10 carbon atoms which optionally have a substituent, or an arylene group or heteroarylene group having 6 to 12 carbon atoms which optionally have a substituent, or a cycloalkylene group or heterocycloalkylene group having 3 to 8 carbon atoms which optionally have a substituent;
L is represented by $-(CH_2)_d-[R_3-(CH_2)_e]_f-$ or $-[(CH_2)_e-R_3]_f-(CH_2)_d-$, and $R_3$ is an oxygen atom, a sulfur atom, -NH-, -NH-(C=O)- or -(C=O)-NH-;
TAG is a tag;
a and c are identical or different and are an integer of 0 to 6,
b is 0 or 1;
d and e are identical or different and are an integer of 0 to 12; and
f is an integer of 0 to 24.

**10.** A method for determining effectiveness of medical intervention about cognitive function, comprising:

first contacting lipoprotein in a blood sample of a subject to whom the medical intervention is not performed with a labeled lipid and measuring the labeled lipid incorporated into the lipoprotein to obtain a first measured value, wherein the first contacting comprises: contacting the lipoprotein incorporating the labeled lipid with a capture body that binds to the lipoprotein to form a complex, and wherein the complex comprises the lipoprotein incorporating the labeled lipid and the capture body;
after the first contacting, second contacting lipoprotein in a blood sample of the subject to whom the medical intervention has been performed with a labeled lipid and measuring the labeled lipid incorporated into the lipoprotein to obtain a second measured value, wherein the second contacting comprises: contacting the lipoprotein incorporating the labeled lipid with a capture body that binds to the lipoprotein to form a complex, and wherein the complex comprises the lipoprotein incorporating the labeled lipid and the capture body; and
comparing the first measured value with the second measured value to determine effectiveness of the medical intervention;
wherein the labeled lipid is labelled cholesterol and the lipoprotein comprises ApoE, and
wherein the capture body is an anti-ApoE antibody.

**11.** The method according to claim 10, wherein, when the second measured value is greater than the first measured value, it is determined that the medical intervention is effective for the subject.

**12.** The method according to claim 10, wherein, when the second measured value is equal to or less than the first measured value, it is determined that the medical intervention is not effective for the subject.

## Patentansprüche

**1.** Verfahren zur Gewinnung von Informationen über die kognitive Funktion eines Subjekts, umfassend: Inkontaktbringen von Lipoprotein in einer Blutprobe eines Subjekts mit einem markierten Lipid; und Messen des markierten Lipids, das in das Lipoprotein aufgenommen wurde, um einen Messwert zu erhalten, wobei der Messwert ein Indikator für eine kognitive Beeinträchtigung ist;

wobei das markierte Lipid ein markiertes Cholesterin ist und das Lipoprotein ApoE umfasst,
wobei das Inkontaktbringen Folgendes umfasst: Inkontaktbringen des Lipoproteins, das das markierte Lipid aufgenommen hat, mit einem Einfangskörper, der an das Lipoprotein bindet, um einen Komplex zu bilden, wobei der Komplex das Lipoprotein, das das markierte Lipid aufgenommen hat, und den Einfangskörper umfasst, und wobei der Einfangskörper ein Anti-ApoE-Antikörper ist.

**2.** Verfahren nach Anspruch 1, wobei die Informationen über die kognitive Funktion Informationen über das Risiko einer Abnahme der kognitiven Funktion des Subjekts und/oder Informationen sind, die einen Zustand der kognitiven Funktion des Subjekts anzeigen.

**3.** Verfahren nach Anspruch 2, wobei, wenn der Messwert gleich oder kleiner als ein vorgegebener Schwellenwert ist, der Messwert nahelegt, dass ein Risiko einer Abnahme der kognitiven Funktion des Subjekts hoch ist und/oder dass die kognitive Funktion des Subjekts abgenommen ist.

**4.** Verfahren nach Anspruch 2, wobei, wenn der Messwert größer als ein vorgegebener Schwellenwert ist, der Messwert nahelegt, dass ein Risiko einer Abnahme der kognitiven Funktion des Subjekts niedrig ist und/oder dass die kognitive Funktion des Subjekts nicht abgenommen ist.

**5.** Verfahren nach Anspruch 1, wobei die Informationen über die kognitive Funktion Informationen über das Auftretensrisiko einer Erkrankung des zentralen Nervensystems und/oder Informationen sind, die ein Vorhandensein oder Nichtvorhandensein einer Erkrankung des zentralen Nervensystems anzeigen.

**6.** Verfahren nach Anspruch 5, wobei, wenn der Messwert gleich oder kleiner als ein vorgegebener Schwellenwert ist, der Messwert nahelegt, dass das Risiko, eine Erkrankung des zentralen Nervensystems zu entwickeln, hoch ist und/oder dass das Subjekt eine Erkrankung des zentralen Nervensystems entwickelt.

**7.** Verfahren nach Anspruch 5, wobei, wenn der Messwert größer als ein vorgegebener Schwellenwert ist, der Messwert nahelegt, dass das Risiko, eine Erkrankung des zentralen Nervensystems zu entwickeln, niedrig ist und/oder dass das Subjekt keine Erkrankung des zentralen Nervensystems entwickelt.

**8.** Verfahren nach einem der Ansprüche 5 bis 7, wobei die Erkrankung des zentralen Nervensystems die Alzheimer-Krankheit ist.

**9.** Verfahren nach Anspruch 1, wobei

das markierte Sterol ein markiertes Cholesterin ist, das durch eine folgende Formel (I) dargestellt ist:

[Chemische Formel 1]

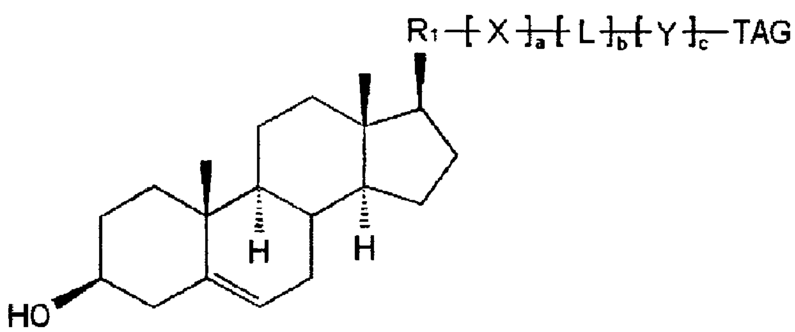

(I)

wobei $R_1$ eine Alkylengruppe mit 1 bis 6 Kohlenstoffatomen ist, die wahlweise eine Methylgruppe aufweist;
X und Y identisch oder unterschiedlich sind, und dargestellt sind durch $-R_2$-NH-, - NH-$R_2$-, $-R_2$-(C=O)-NH-,

-(C=O)-NH-$R_2$-, -$R_2$-NH-(C=O)-, -NH-(C=O)-$R_2$-, -$R_2$-(C=O)-, - (C=O)-$R_2$-, -$R_2$-(C=O)-O-, -(C=O)-O-$R_2$-, -$R_2$-O-(C=O)-, -O-(C=O)-$R_2$-, -$R_2$-(C=S)-NH-, - (C=S)-NH-$R_2$-, -$R_2$-NH-(C=S)-, -NH-(C=S)-$R_2$-, -$R_2$-O-, -O-$R_2$-, -$R_2$-S-, oder -S-$R_2$-, und jedes $R_2$ unabhängig eine atomare Bindung, eine Alkylengruppe mit 1 bis 10 Kohlenstoffatomen, die wahlweise einen Substituenten aufweist, oder eine Arylengruppe oder Heteroarylengruppe mit 6 bis 12 Kohlenstoffatomen, die wahlweise einen Substituenten aufweist, oder eine Cycloalkylengruppe oder Heterocycloalkylengruppe mit 3 bis 8 Kohlenstoffatomen, die wahlweise einen Substituenten aufweist, ist;

L dargestellt ist durch $-(CH_2)_d-[R_3-(CH_2)_e]_f-$ oder $-[(CH_2)_e-R_3]_f-(CH_2)_d-$, und $R_3$ ist ein Sauerstoffatom, ein Schwefelatom, -NH-, -NH-(C=O)- oder -(C=O)-NH-;

TAG ein Tag ist;

a und c identisch oder unterschiedlich sind und eine ganze Zahl von 0 bis 6 sind,

b 0 oder 1 ist;

d und e identisch oder unterschiedlich sind und eine ganze Zahl von 0 bis 12 sind; und

f eine ganze Zahl von 0 bis 24 ist.

**10.** Verfahren zum Bestimmen der Wirksamkeit der medizinischen Intervention in Bezug auf die kognitive Funktion, umfassend:

erstes Inkontaktbringen von Lipoprotein in einer Blutprobe eines Subjekts, bei dem die medizinische Intervention nicht durchgeführt wird, mit einem markierten Lipid und Messen des markierten Lipids, das in das Lipoprotein aufgenommen wurde, um einen ersten Messwert zu erhalten, wobei das erste Inkontaktbringen Folgendes umfasst: Inkontaktbringen des Lipoproteins, das das markierte Lipid aufgenommen hat, mit einem Einfangskörper, der an das Lipoprotein bindet, um einen Komplex zu bilden, und wobei der Komplex das Lipoprotein, das das markierte Lipid aufgenommen hat, und den Einfangskörper umfasst;

nach dem ersten Inkontaktbringen, zweites Inkontaktbringen von Lipoprotein in einer Blutprobe des Subjekts, bei dem die medizinische Intervention durchgeführt worden ist, mit einem markierten Lipid und Messen des markierten Lipids, das in das Lipoprotein aufgenommen wurde, um einen zweiten Messwert zu erhalten, wobei das zweite Inkontaktbringen Folgendes umfasst: Inkontaktbringen des Lipoproteins, das das markierte Lipid aufgenommen hat, mit einem Einfangskörper, der an das Lipoprotein bindet, um einen Komplex zu bilden, und wobei der Komplex das Lipoprotein, das das markierte Lipid aufgenommen hat, und den Einfangskörper umfasst; und

Vergleichen des ersten Messwerts mit dem zweiten Messwert, um die Wirksamkeit der medizinischen Intervention zu bestimmen;

wobei das markierte Lipid markiertes Cholesterin ist und das Lipoprotein ApoE umfasst, und

wobei der Einfangskörper ein Anti-ApoE-Antikörper ist.

**11.** Verfahren nach Anspruch 10, wobei, wenn der zweite Messwert größer als der erste Messwert ist, bestimmt wird, dass die medizinische Intervention für das Subjekt wirksam ist.

**12.** Verfahren nach Anspruch 10, wobei, wenn der zweite Messwert gleich oder kleiner als der erste Messwert ist, bestimmt wird, dass die medizinische Intervention für das Subjekt nicht wirksam ist.

## Revendications

**1.** Procédé permettant d’obtenir des informations sur la fonction cognitive d’un sujet, comprenant : une mise en contact d’une lipoprotéine dans un échantillon de sang d’un sujet avec un lipide marqué ; et une mesure du lipide marqué incorporé dans la lipoprotéine de manière à obtenir une valeur mesurée, dans lequel la valeur mesurée est un indicateur de trouble cognitif ;

dans lequel le lipide marqué est du cholestérol marqué et la lipoprotéine comprend ApoE,

dans lequel la mise en contact comprend : une mise en contact de la lipoprotéine incorporant le lipide marqué avec un corps de capture qui se lie à la lipoprotéine de façon à former un complexe, dans lequel le complexe comprend la lipoprotéine incorporant le lipide marqué et le corps de capture, et

dans lequel le corps de capture est un anticorps anti-ApoE.

**2.** Procédé selon la revendication 1, dans lequel les informations sur la fonction cognitive sont des informations sur le risque de déclin de la fonction cognitive du sujet et/ou des informations indiquant un état de la fonction cognitive du

sujet.

**3.** Procédé selon la revendication 2, dans lequel, lorsque la valeur mesurée est inférieure ou égale à un seuil prédéterminé, la valeur mesurée suggère qu'un risque de déclin de la fonction cognitive du sujet est élevé et/ou que la fonction cognitive du sujet est diminuée.

**4.** Procédé selon la revendication 2, dans lequel, lorsque la valeur mesurée est supérieure à un seuil prédéterminé, la valeur mesurée suggère qu'un risque de déclin de la fonction cognitive du sujet est faible et/ou que la fonction cognitive du sujet n'est pas diminuée.

**5.** Procédé selon la revendication 1, dans lequel les informations sur la fonction cognitive sont des informations sur un risque de survenue d'une maladie du système nerveux central et/ou des informations indiquant une présence ou absence d'une maladie du système nerveux central.

**6.** Procédé selon la revendication 5, dans lequel, lorsque la valeur mesurée est inférieure ou égale à un seuil prédéterminé, la valeur mesurée suggère que le risque de développer une maladie du système nerveux central est élevé et/ou que le sujet développe une maladie du système nerveux central.

**7.** Procédé selon la revendication 5, dans lequel, lorsque la valeur mesurée est supérieure à un seuil prédéterminé, la valeur mesurée suggère que le risque de développer une maladie du système nerveux central est faible et/ou que le sujet ne développe pas une maladie du système nerveux central.

**8.** Procédé selon l'une quelconque des revendications 5 à 7, dans lequel la maladie du système nerveux central est la maladie d'Alzheimer.

**9.** Procédé selon la revendication 1, dans lequel

le stérol marqué est du cholestérol marqué représenté par la formule suivante (I) :

[Formule Chimique 1]

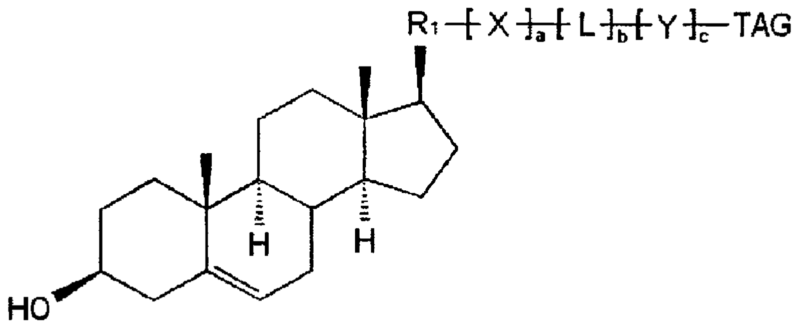

(I)

dans lequel $R_1$ est un groupe alkylène présentant de 1 à 6 atomes de carbone qui présentent facultativement un groupe méthyle ;
X et Y sont identiques ou différents, et sont représentés par $-R_2-NH-$, $-NH-R_2-$, $-R_2-(C{=}O)-NH-$, $-(C{=}O)-NH-R_2-$, $-R_2-NH-(C{=}O)-$, $-NH-(C{=}O)-R_2-$, $-R_2-(C{=}O)-$, $-(C{=}O)-R_2-$, $-R_2-(C{=}O)-O-$, $-(C{=}O)-O-R_2-$, $-R_2-O-(C{=}O)-$, $-O-(C{=}O)-R_2-$, $-R_2-(C{=}S)-NH-$, $-(C{=}S)-NH-R_2-$, $-R_2-NH-(C{=}S)-$, $-NH-(C{=}S)-R_2-$, $-R_2-O-$, $-O-R_2-$, $-R_2-S-$, ou $-S-R_2-$, et chaque $R_2$ est indépendamment une liaison atomique, un groupe alkylène présentant de 1 à 10 atomes de carbone qui présentent facultativement un substituant, ou un groupe arylène ou un groupe hétéroarylène présentant de 6 à 12 atomes de carbone qui présentent facultativement un substituant, ou un groupe cycloalkylène ou un groupe hétérocycloalkylène présentant de 3 à 8 atomes de carbone qui présentent facultativement un substituant ;
L est représenté par $-(CH_2)_d-[R_3-(CH_2)_e]_f-$ ou $-[(CH_2)_e-R_3]_f-(CH_2)_d-$, et $R_3$ est un atome d'oxygène, un atome de soufre, -NH-, -NH-(C=O)- ou -(C=O)-NH- ;
TAG est un marqueur ;
a et c sont identiques ou différents et sont un nombre entier de 0 à 6,

b est 0 ou 1 ;
d et e sont identiques ou différents et sont un nombre entier de 0 à 12 ; et
f est un nombre entier de 0 à 24.

**10.** Procédé de détermination de l'efficacité d'une intervention médicale concernant la fonction cognitive, comprenant :

une première mise en contact d'une lipoprotéine dans un échantillon de sang d'un sujet auquel l'intervention médicale n'est pas effectuée avec un lipide marqué et une mesure du lipide marqué incorporé dans la lipoprotéine de manière à obtenir une première valeur mesurée, dans lequel la première mise en contact comprend : une mise en contact de la lipoprotéine incorporant le lipide marqué avec un corps de capture qui se lie à la lipoprotéine de façon à former un complexe, et dans lequel le complexe comprend la lipoprotéine incorporant le lipide marqué et le corps de capture ;
après la première mise en contact, une deuxième mise en contact d'une lipoprotéine dans un échantillon de sang du sujet auquel l'intervention médicale a été effectuée avec un lipide marqué et une mesure du lipide marqué incorporé dans la lipoprotéine de manière à obtenir une deuxième valeur mesurée, dans lequel la deuxième mise en contact comprend : une mise en contact de la lipoprotéine incorporant le lipide marqué avec un corps de capture qui se lie à la lipoprotéine de façon à former un complexe, et dans lequel le complexe comprend la lipoprotéine incorporant le lipide marqué et le corps de capture ; et
une comparaison de la première valeur mesurée à la deuxième valeur mesurée afin de déterminer l'efficacité de l'intervention médicale ;
dans lequel le lipide marqué est du cholestérol marqué et la lipoprotéine comprend ApoE, et
dans lequel le corps de capture est un anticorps anti-ApoE.

**11.** Procédé selon la revendication 10, dans lequel, lorsque la deuxième valeur mesurée est supérieure à la première valeur mesurée, il est déterminé que l'intervention médicale est efficace pour le sujet.

**12.** Procédé selon la revendication 10, dans lequel, lorsque la deuxième valeur mesurée est inférieure ou égale à la première valeur mesurée, il est déterminé que l'intervention médicale n'est pas efficace pour le sujet.

FIG. 1A

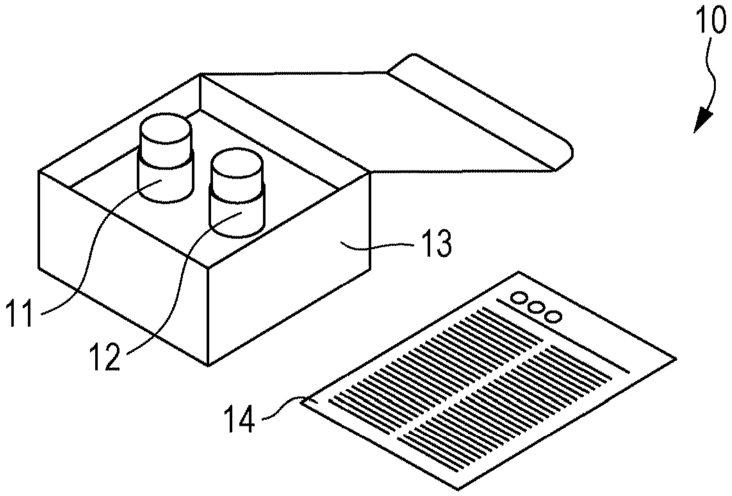


FIG. 1B

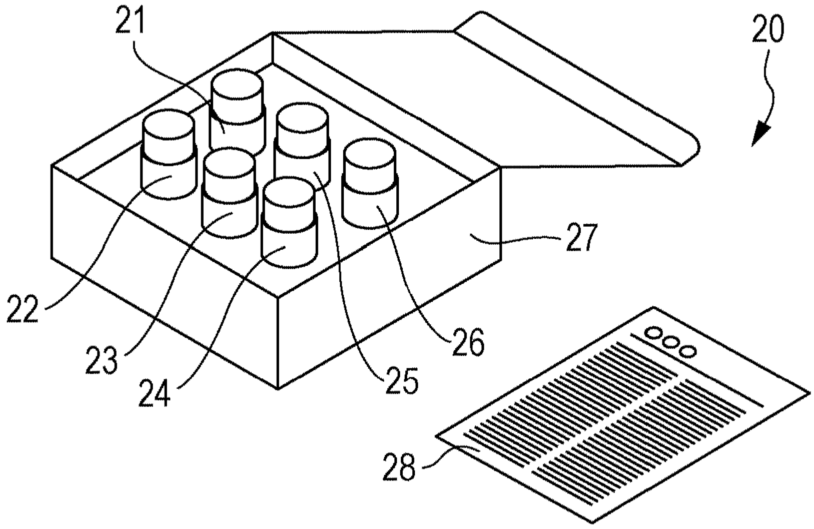

FIG. 2

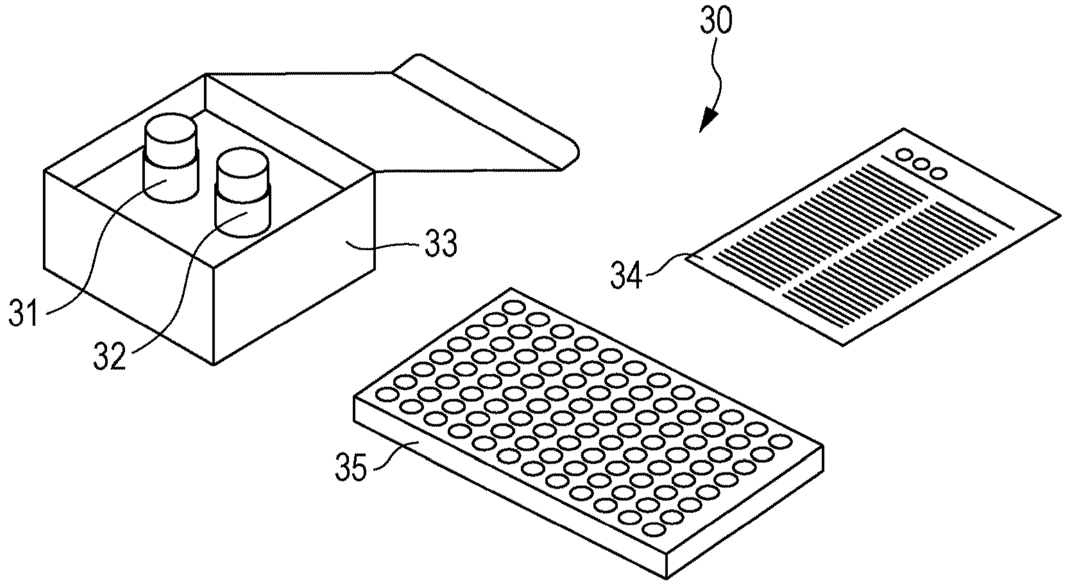


FIG. 3

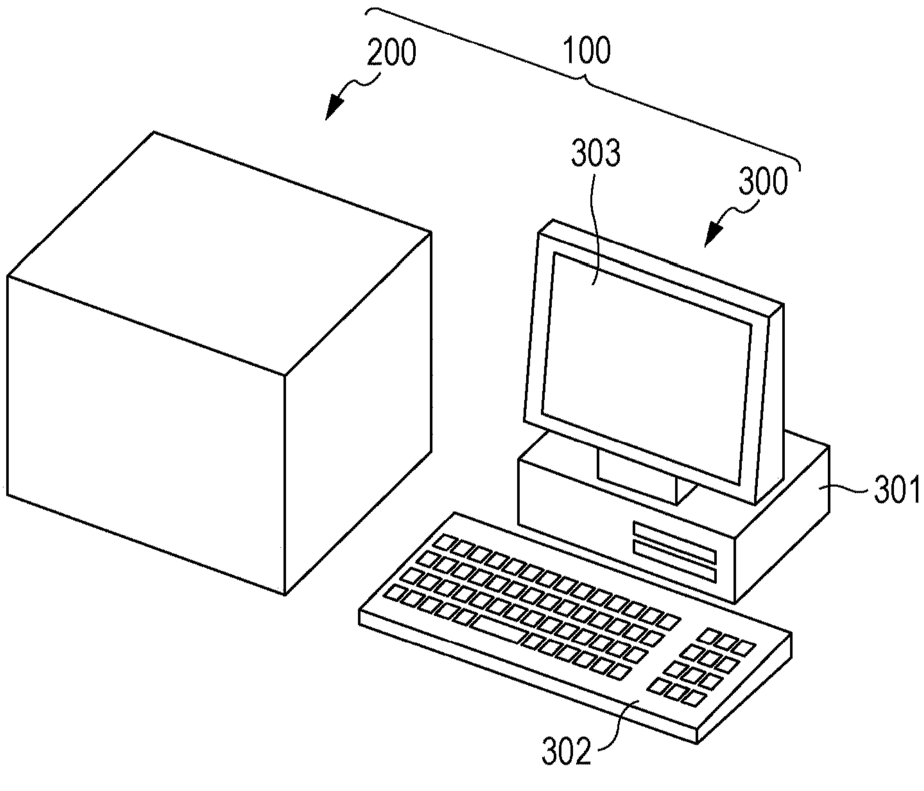

*FIG. 4*

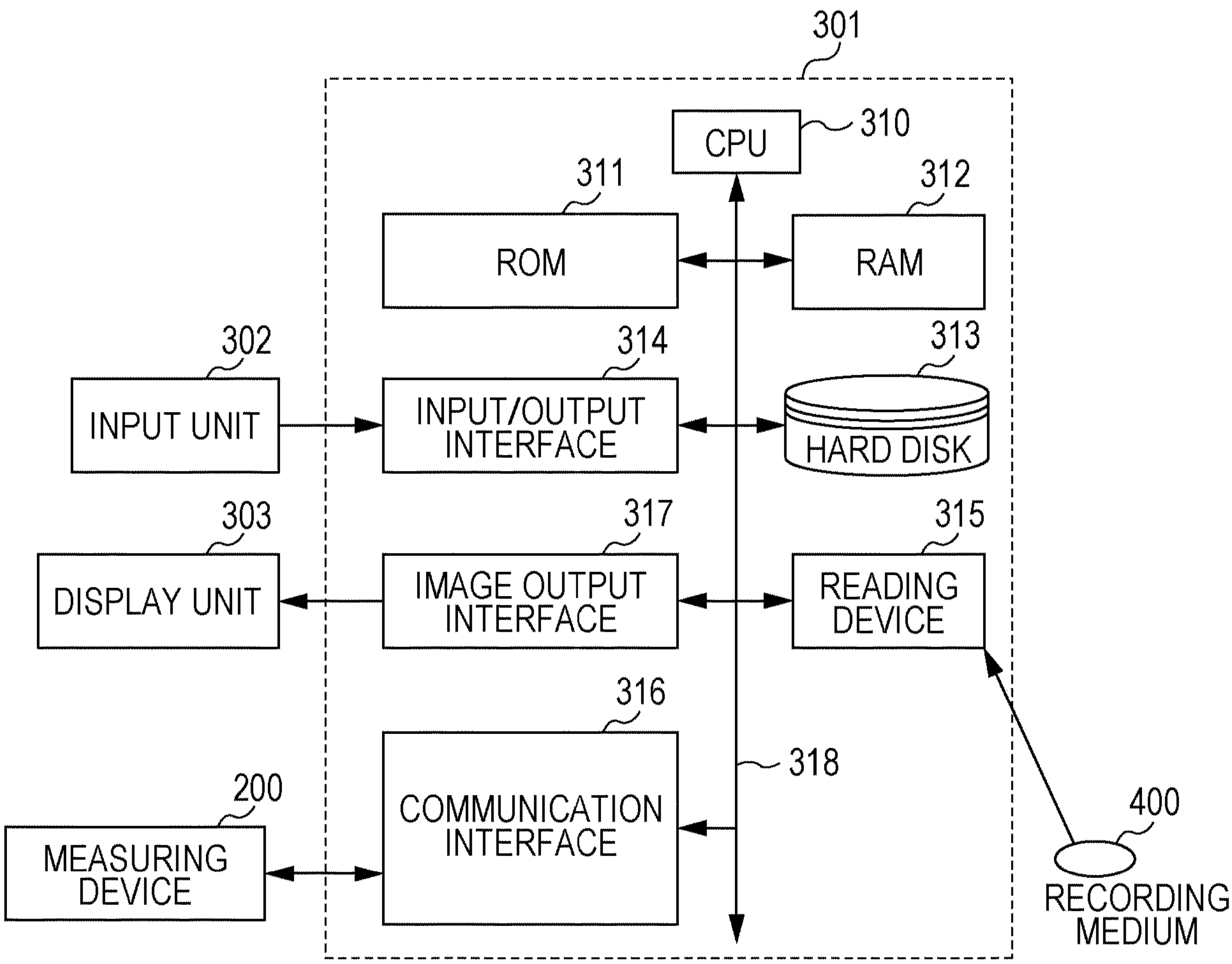
301
CPU
310
311
312
ROM
RAM
302
314
313
INPUT UNIT
INPUT/OUTPUT INTERFACE
HARD DISK
303
317
315
DISPLAY UNIT
IMAGE OUTPUT INTERFACE
READING DEVICE
316
318
200
400
MEASURING DEVICE
COMMUNICATION INTERFACE
RECORDING MEDIUM

FIG. 5A

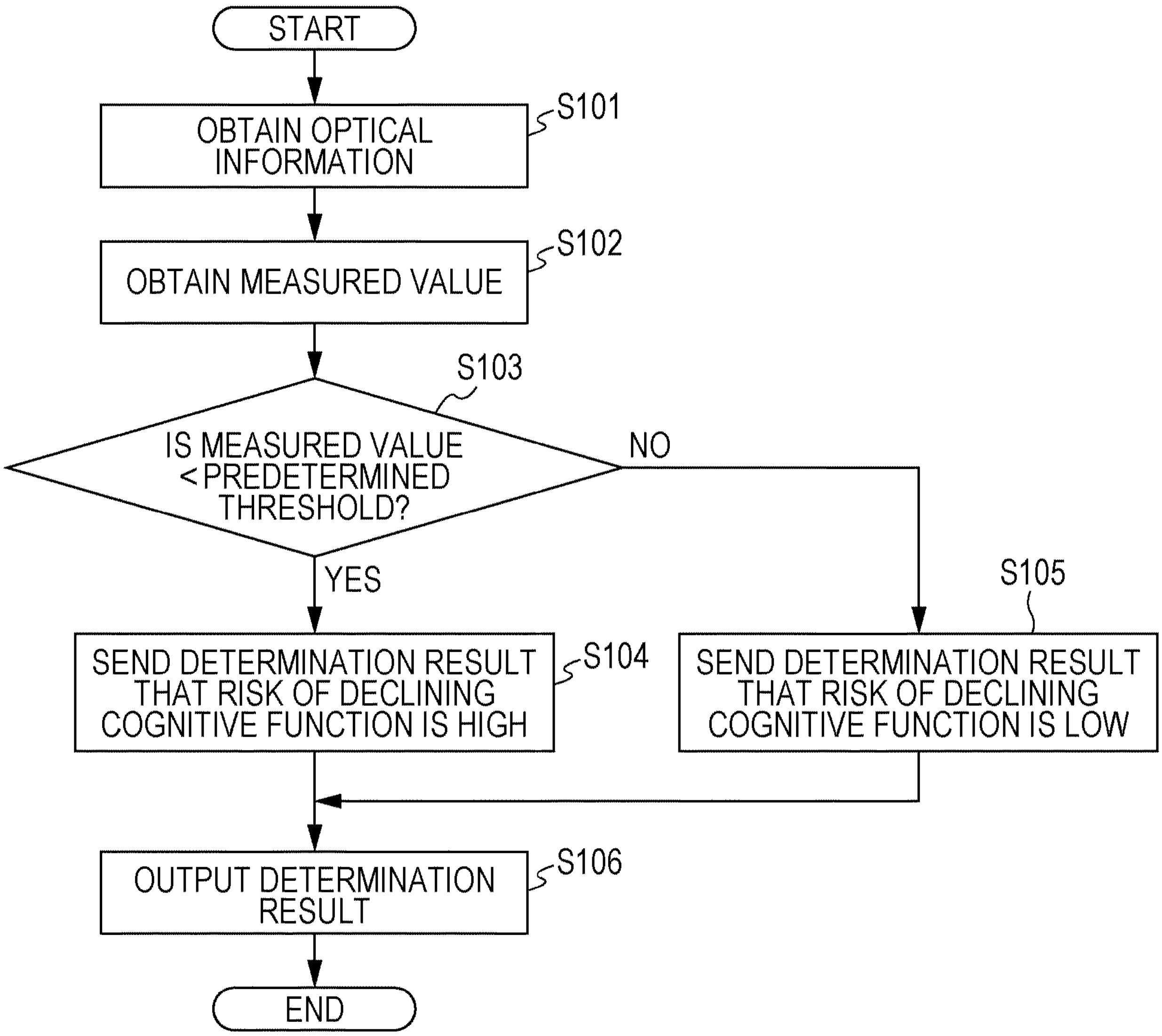
START
OBTAIN OPTICAL INFORMATION
S101
OBTAIN MEASURED VALUE
S102
S103
IS MEASURED VALUE <PREDETERMINED THRESHOLD?
NO
YES
S105
SEND DETERMINATION RESULT THAT RISK OF DECLINING COGNITIVE FUNCTION IS HIGH
S104
SEND DETERMINATION RESULT THAT RISK OF DECLINING COGNITIVE FUNCTION IS LOW
OUTPUT DETERMINATION RESULT
S106
END

FIG. 5B

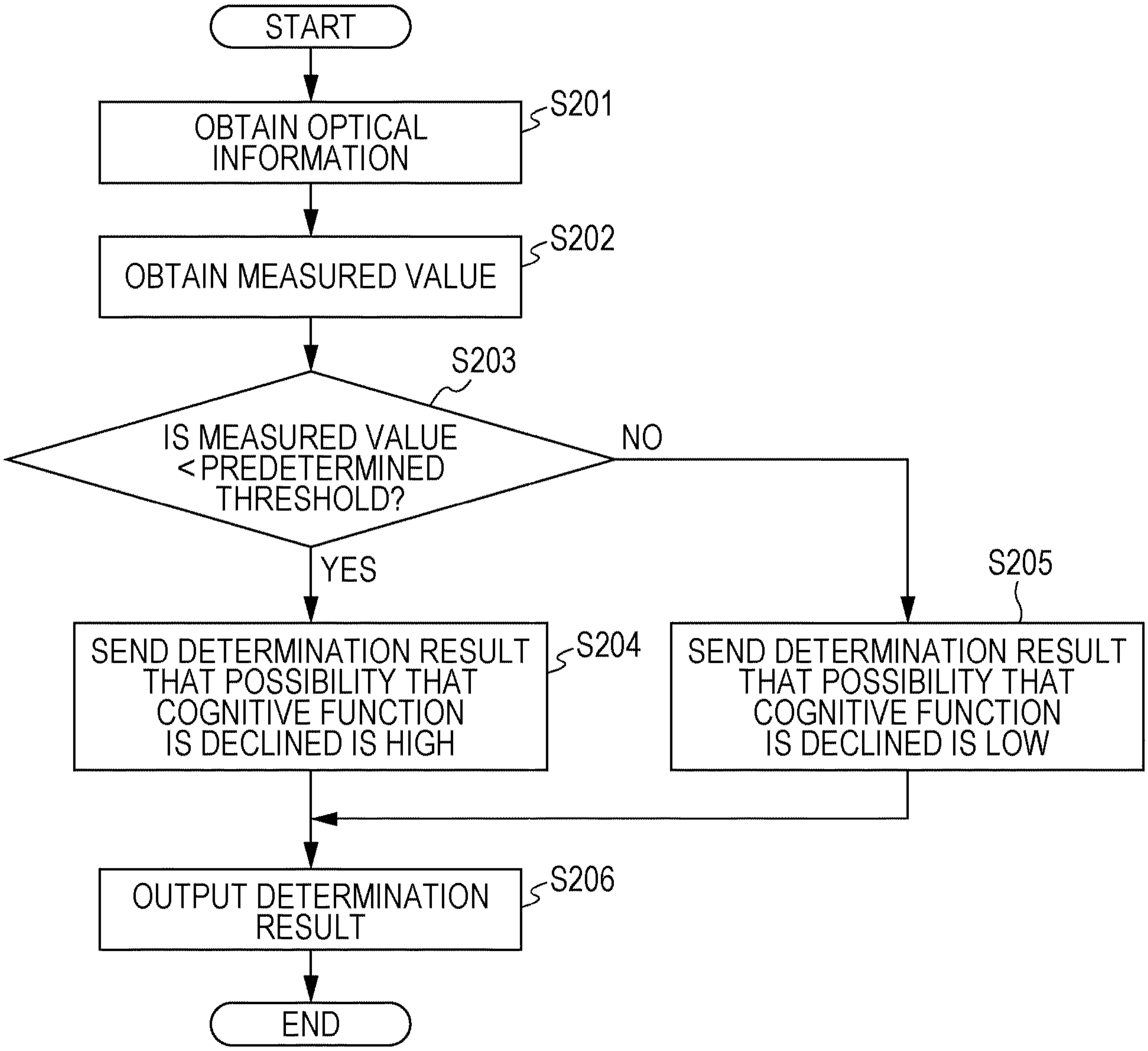
START
OBTAIN OPTICAL INFORMATION
S201
OBTAIN MEASURED VALUE
S202
S203
IS MEASURED VALUE <PREDETERMINED THRESHOLD?
NO
YES
S205
SEND DETERMINATION RESULT THAT POSSIBILITY THAT COGNITIVE FUNCTION IS DECLINED IS HIGH
S204
SEND DETERMINATION RESULT THAT POSSIBILITY THAT COGNITIVE FUNCTION IS DECLINED IS LOW
OUTPUT DETERMINATION RESULT
S206
END

FIG. 5C

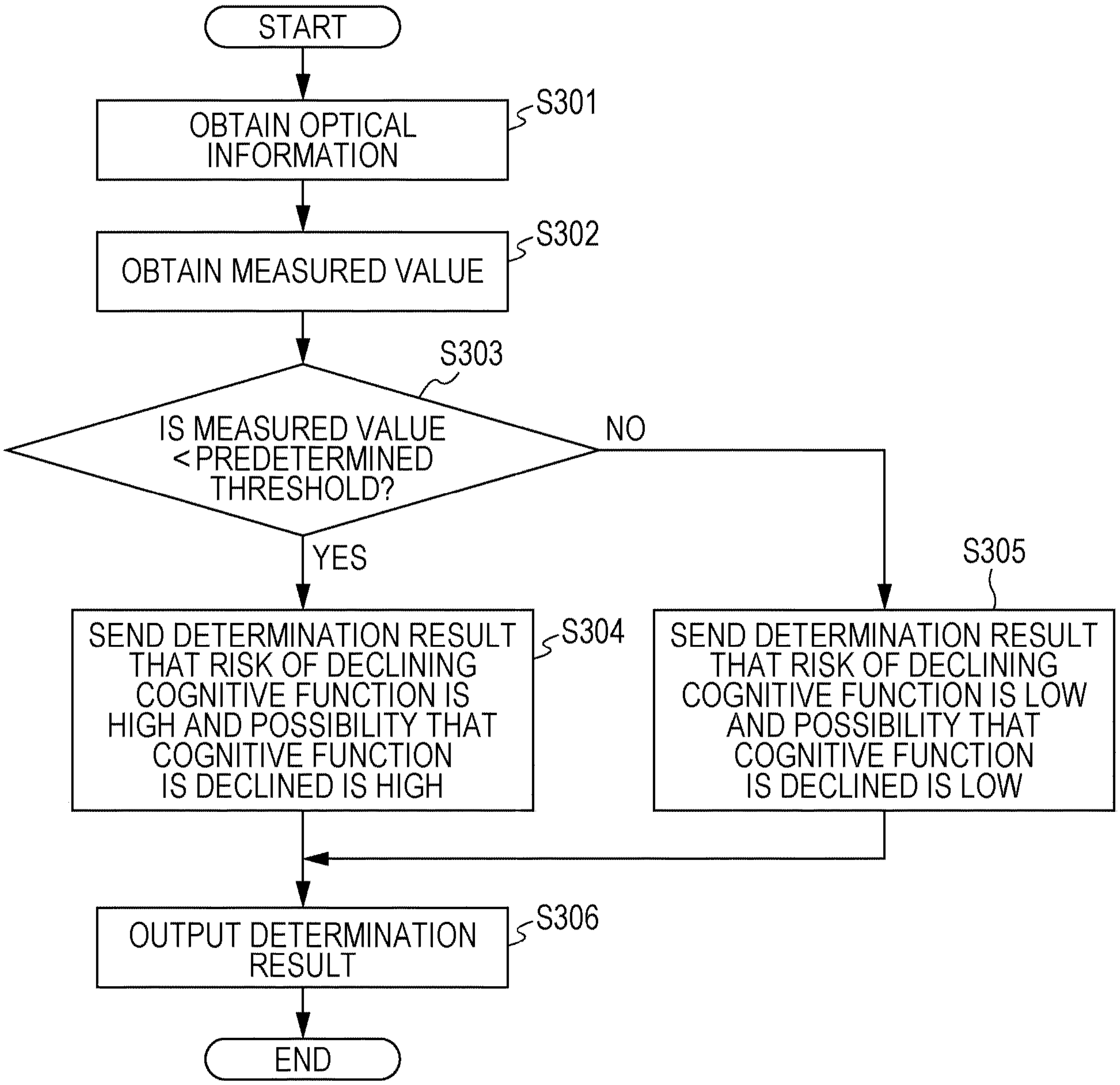
START
OBTAIN OPTICAL INFORMATION
S301
OBTAIN MEASURED VALUE
S302
S303
IS MEASURED VALUE < PREDETERMINED THRESHOLD?
NO
YES
S305
SEND DETERMINATION RESULT THAT RISK OF DECLINING COGNITIVE FUNCTION IS HIGH AND POSSIBILITY THAT COGNITIVE FUNCTION IS DECLINED IS HIGH
S304
SEND DETERMINATION RESULT THAT RISK OF DECLINING COGNITIVE FUNCTION IS LOW AND POSSIBILITY THAT COGNITIVE FUNCTION IS DECLINED IS LOW
OUTPUT DETERMINATION RESULT
S306
END

FIG. 6

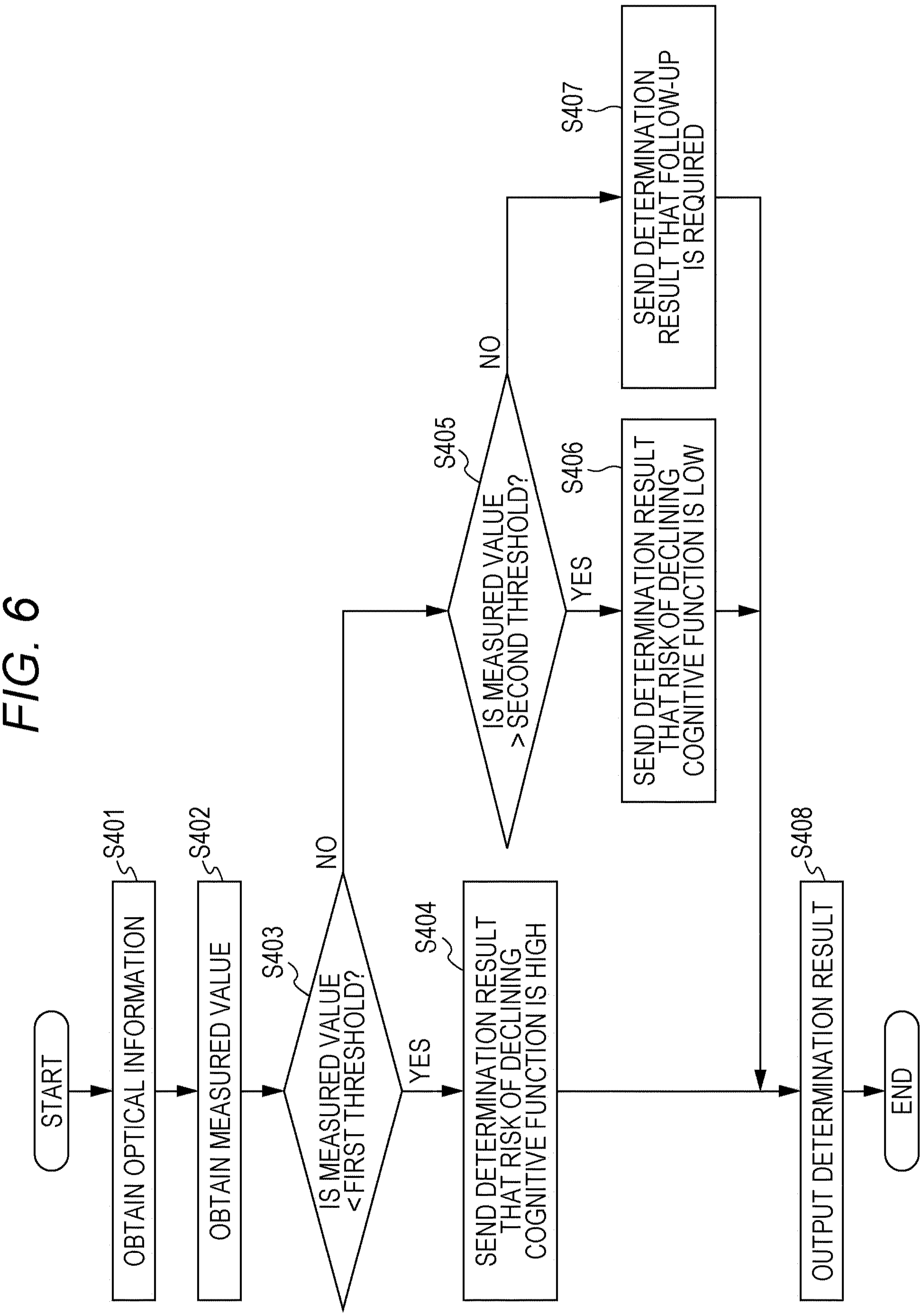
START
OBTAIN OPTICAL INFORMATION
S401
OBTAIN MEASURED VALUE
S402
IS MEASURED VALUE <FIRST THRESHOLD?
S403
YES
NO
SEND DETERMINATION RESULT THAT RISK OF DECLINING COGNITIVE FUNCTION IS HIGH
S404
IS MEASURED VALUE >SECOND THRESHOLD?
S405
YES
NO
SEND DETERMINATION RESULT THAT RISK OF DECLINING COGNITIVE FUNCTION IS LOW
S406
SEND DETERMINATION RESULT THAT FOLLOW-UP IS REQUIRED
S407
OUTPUT DETERMINATION RESULT
S408
END

FIG. 7

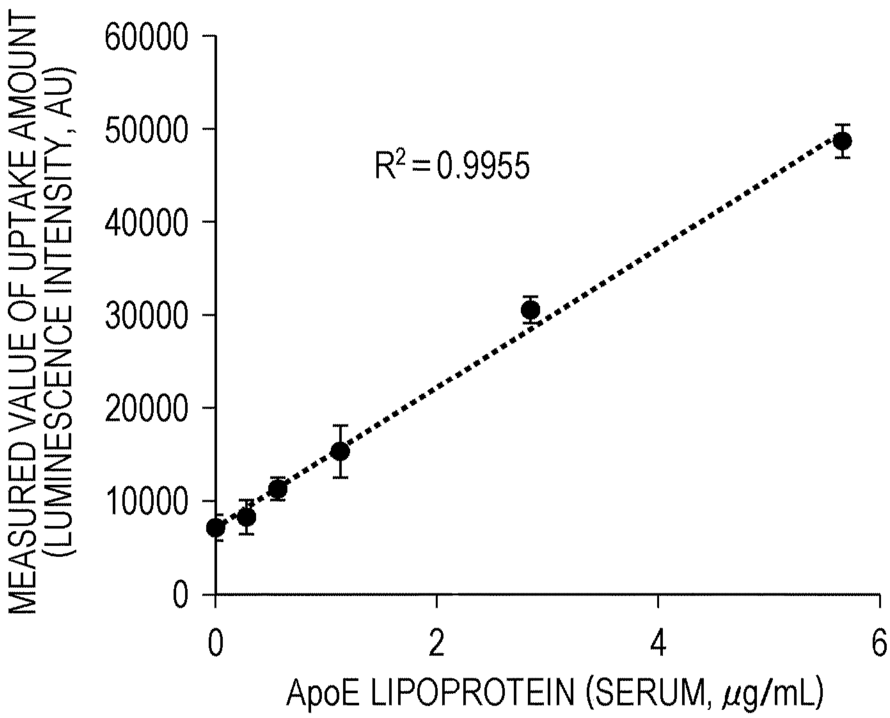
MEASURED VALUE OF UPTAKE AMOUNT (LUMINESCENCE INTENSITY, AU)
60000
50000
40000
30000
20000
10000
0
$R^2 = 0.9955$
0
2
4
6
ApoE LIPOPROTEIN (SERUM, μg/mL)

FIG. 8

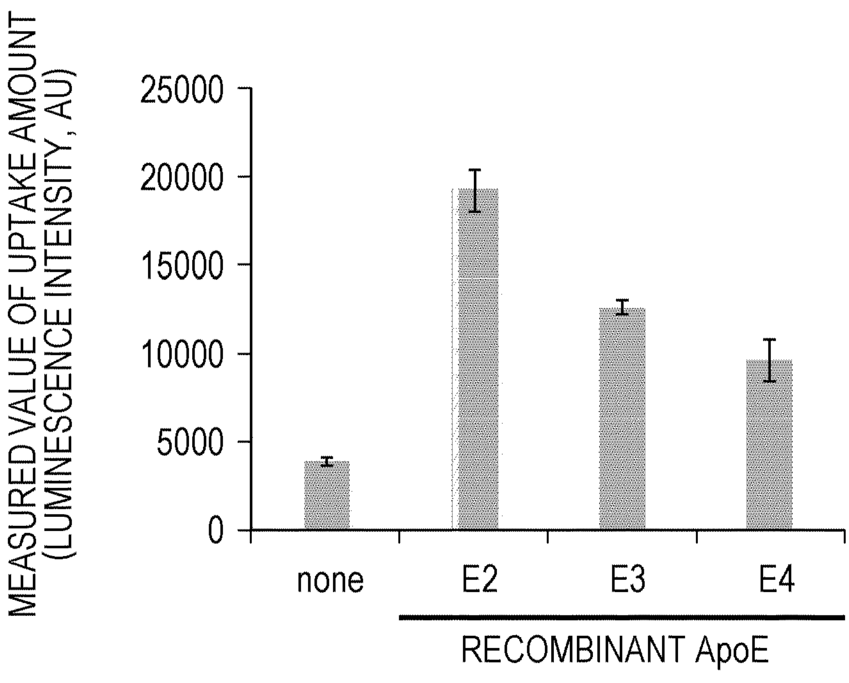
MEASURED VALUE OF UPTAKE AMOUNT (LUMINESCENCE INTENSITY, AU)
25000
20000
15000
10000
5000
0
none
E2
E3
E4
RECOMBINANT ApoE

*FIG. 9*

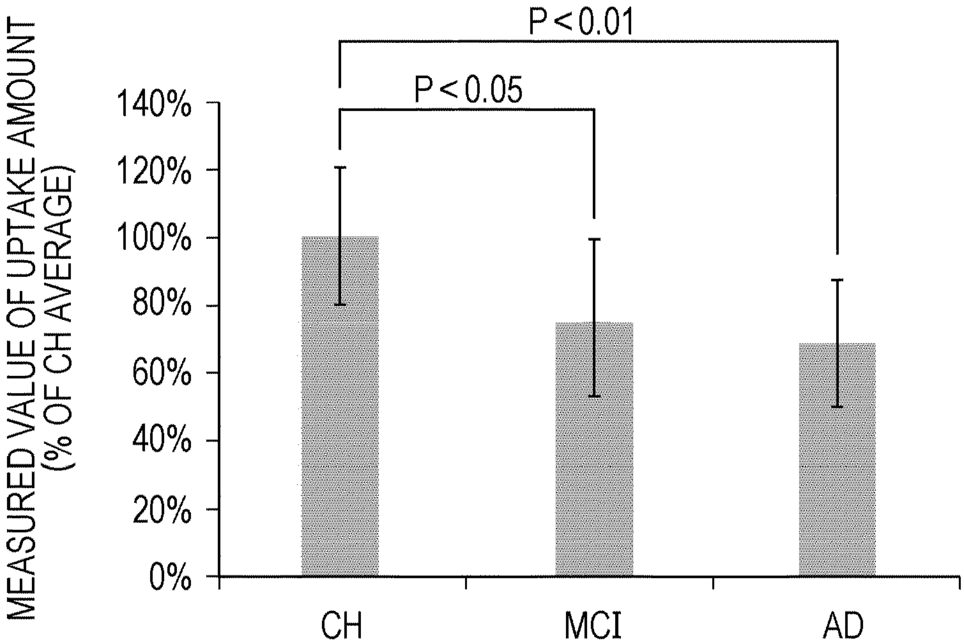
P<0.01
P<0.05
MEASURED VALUE OF UPTAKE AMOUNT
(% OF CH AVERAGE)
140%
120%
100%
80%
60%
40%
20%
0%
CH
MCI
AD

*FIG. 10*

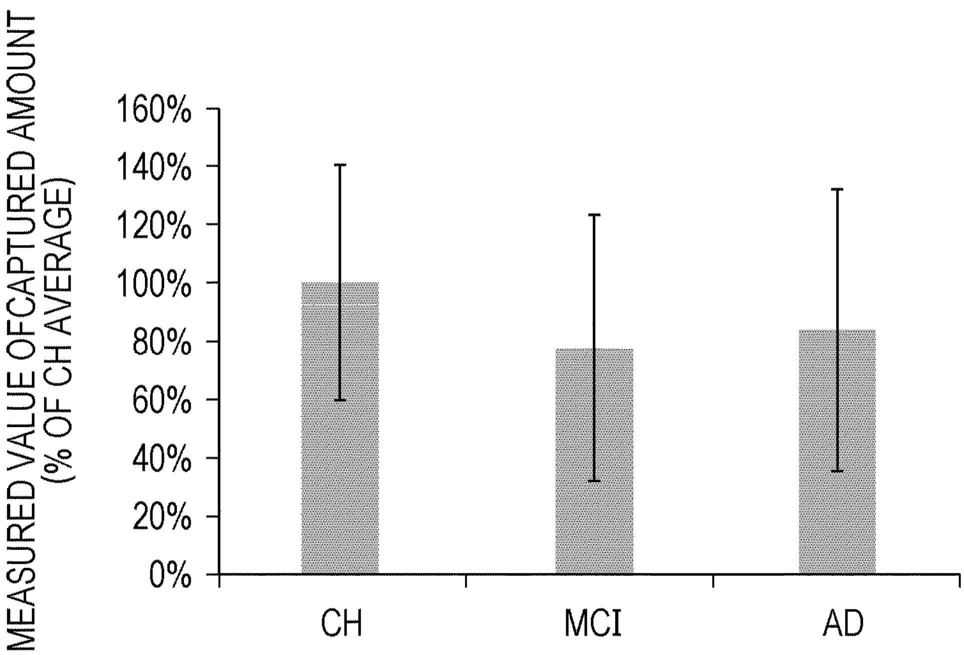
MEASURED VALUE OFCAPTURED AMOUNT
(% OF CH AVERAGE)
160%
140%
120%
100%
80%
60%
40%
20%
0%
CH
MCI
AD

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 10809249 B2
- US 20170315112 A1
- US 20170315112 A


### Non-patent literature cited in the description


- **YASSINE H.N. et al.** ABCA 1-Mediated Cholesterol Efflux Capacity to Cerebrospinal Fluid Is Reduced in Patients With Mild Cognitive Impairment and Alzheimer's Disease. *J Am Heart Assoc.*, 12 February 2016, vol. 5 (2)
- Cholesterol Uptake Capacity: A New Measure of HDL Functionality for Coronary Risk Assessment. **AMANE HARADA et al.** The Journal of Applied Laboratory Medicine. An AACC Publication, vol. 2, 186-200
- **KHALIL ABDELOUAHED et al.** Impairment of the ABCA1 and SR-BI-mediated cholesterol afflux pathways and HDL anti-inflammatory activity in Alzheimer's disease. *echanisms of Ageing and Development*, vol. 133 (1), 20-29
- **NAGANO et al.** *J Am Heart Assoc.*, 07 May 2019, vol. 8 (9)
- **MCKHANN et al.** *Alzheimer's & Dementia*, 2011, vol. 7, 263-269
- **EDWARD B. N. et al.** *Biology*, 2019, vol. 8, 53
- **PITTO-BARRY A.** ; **BARRY N.P.E.** *Poly. Chem*, 2014, vol. 5, 3291-3297
- *J Neurochem.*, March 2000, vol. 74 (3), 1008-16